(19) Europäisches Patentamt
European Patent Office
Office européen des brevets

(11) EP 2 164 880 B1

(12) **EUROPÄISCHE PATENTSCHRIFT**

(45) Veröffentlichungstag und Bekanntmachung des Hinweises auf die Patenterteilung:
**06.06.2012 Patentblatt 2012/23**

(21) Anmeldenummer: **08774394.4**

(22) Anmeldetag: **27.06.2008**

(51) Int Cl.:
***C08F 265/06*** (2006.01) ***C08F 285/00*** (2006.01)
***C09D 151/00*** (2006.01) ***C09J 151/00*** (2006.01)

(86) Internationale Anmeldenummer:
**PCT/EP2008/058223**

(87) Internationale Veröffentlichungsnummer:
**WO 2009/007254 (15.01.2009 Gazette 2009/03)**

(54) **EMULSIONSPOLYMERISAT ENTHALTEND AKTIVATOREN, VERFAHREN ZU DESSEN HERSTELLUNG SOWIE DESSEN VERWENDUNG IN ZWEI- ODER MEHRKOMPONENTENSYSTEMEN**

EMULSION POLYMER COMPRISING ACTIVATORS, PROCESS FOR PREPARATION THEREOF AND USE THEREOF IN TWO-COMPONENT OR MULTICOMPONENT SYSTEMS

POLYMÈRE EN ÉMULSION CONTENANT DES ACTIVATEURS, SON PROCÉDÉ DE FABRICATION ET SON UTILISATION DANS DES SYSTÈMES À DEUX COMPOSANTS OU PLUS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.07.2007 DE 102007032836**

(43) Veröffentlichungstag der Anmeldung:
**24.03.2010 Patentblatt 2010/12**

(73) Patentinhaber: **Evonik Röhm GmbH**
**64293 Darmstadt (DE)**

(72) Erfinder:
- **SCHMITT, Gerold**
**63743 Aschaffenburg (DE)**
- **KLESSE, Wolfgang**
**55127 Mainz (DE)**
- **KNEBEL, Joachim**
**64665 Alsbach-Hähnlein (DE)**

(56) Entgegenhaltungen:
**DE-A1- 2 361 501 DE-A1- 10 339 329**
**US-A- 3 886 116**

- **J. DNEBOSKY: "Polymerizable Amines as Promoters of Cold-Curing Resins and Composites" J. DENT. RES., Bd. 54, Nr. 4, 1975, Seiten 772-776, XP002499007**
- **DATABASE WPI Week 198346 Thomson Scientific, London, GB; AN 1983-817514 XP002499008 & JP 58 170737 A (LION CORP) 7. Oktober 1983 (1983-10-07)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### 1. Gebiet der Erfindung

Die Erfindung beschreibt ein Emulsionspolymerisat mit polymerfixierten Aktivatoren sowie ein Verfahren zu dessen Herstellung. Die Erfindung betrifft auch ein durch ein Redoxinitiatorsystem härtendes Zwei- oder Mehrkomponenten-System mit steuerbarer Topfzeit aufweisend das Emulsionspolymerisat mit polymerfixiertem Aktivator sowie ein ethylenisch ungesättigtes Monomer oder eine Monomermischung aus ethylenisch ungesättigten Monomeren, wobei sowohl das Emulsionspolymerisat als auch das Monomer oder die Monomermischung eine der Komponenten eines Redoxinitiatorsystems enthalten können. Schließlich bezieht sich die Erfindung auch auf die Verwendung der Zwei- oder Mehrkomponentensysteme.

### 2. Stand der Technik

Durch Redoxinitiierung härtende Zweikomponentensysteme auf Basis von radikalisch polymerisierbaren Monomeren sind seit langem bekannt. In der Regel geht man so vor, dass einem flüssigen Monomer oder Monomergemisch, das eine Redoxkomponente enthalten kann, vor der Anwendung die fehlenden Redoxsystemkomponenten bzw. alle Redoxsystemkomponenten zugesetzt werden.

Daneben werden Systeme beschrieben, die zusätzlich ein im Monomer oder Monomergemisch gelöstes Polymer enthalten. Vor allem von Dentalanwendungen sind weiterhin Systeme bekannt, bei denen flüssiges Monomer, ein Perlpolymerisat und ein Redoxinitiatorsystem vor der Anwendung zu einer hochviskosen Masse gemischt werden.

Beispielhaft für eine Vielzahl von Veröffentlichungen zum Thema seien die DE 43 15 788, DE 15 44 924 und die DE 27 10 548 genannt. Allen diesen Systemen wohnt der Nachteil inne, dass nach dem Zusammenmischen der Komponenten die für die Verarbeitung zur Verfügung stehende Zeit (Topfzeit) beschränkt ist, oder dass bei der Applikation Energie, beispielsweise in Form von Mahl- und Reibkräften eingebracht werden muss. Zwar lässt sich durch Reduzierung der Redoxkomponentenkonzentration die Topfzeit bedingt verlängern, dem sind jedoch Grenzen gesetzt, da mit sinkender Redoxkomponentenkonzentration die Aushärtung beeinträchtigt wird. Ein weiterer Nachteil der Formulierungen aus dem Stand der Technik besteht darin, dass die maximalen Konzentrationen am Arbeitsplatz (MAK-Werte) von leichtflüchtigen Monomeren, wie beispielsweise Methacrylsäuremethylester, überschritten werden können. Diesem anwendungstechnischen Nachteil kann man nur begrenzt durch die Verwendung von schwerer flüchtigen Monomeren begegnen, da die beispielsweise häufig eingesetzten Perlpolymerisate durch schwerer flüchtigen Monomere nicht mit ausreichender Geschwindigkeit angequollen werden. Ferner ist die Sauerstoffinhibierung der Polymerisation beim Einsatz der schwerer flüchtigen Monomeren stärker ausgeprägt als bei der Verwendung von Methacrylsäuremethylester.

DE 100 51 762 stellt Monomer-Polymersysteme auf Basis wäßriger Dispersionen zur Verfügung, die neben guten mechanischen Eigenschaften den Vorteil bieten, keine oder nur sehr wenig Monomere zu emittieren und darüber hinaus einfach handhabbar zu sein und über eine hohe Lagerstabilität zu verfügen. Hierzu werden Mischungen von wäßrigen Dispersionen verwendet, deren Teilchen mit einem ethylenisch ungesättigten Monomer angequollen worden sind, das jeweils eine der Redoxkomponenten enthielt. Diese angequollenen wäßrigen Systeme sind praktisch unbegrenzt lagerstabil und härten erst nach Verdunsten des Wassers und der nachfolgenden Filmbildung aus. Der Nachteil dieser Systeme ist, dass die Aushärtung durch die erforderliche Verdunstung des Wassers, insbesondere bei dickeren Schichten, lange dauert und größere Anteile Wasser bei einer Reihe von Anwendungen wie Reaktivklebern stören.

WO 99/15592 beschreibt Reaktiv-Plastisole, die nach thermischer Gelierung und Aushärtung zu Filmen mit guten mechanischen Eigenschaften führen. Diese Plastisole bestehen aus einem bekannten Basispolymerisat, vorzugsweise in Form eines sprühgetrockneten Emulsionspolymerisats, einem reaktiven Monomeranteil, bestehend aus mindestens einem monofunktionellen (Meth)acrylatmonomeren, einem Weichmacher sowie gegebenenfalls weiteren vernetzenden Monomeren, Füllstoffen, Pigmenten und Hilfsstoffen. Das Basispolymerisat kann einen Kern/Schale-Aufbau haben und von 0 - 20 % an polaren Comonomeren enthalten. Die Plastisole sind über Wochen lagerstabil und müssen zur Verfilmung auf hohe Temperaturen (z. B. 130 °C) erhitzt werden.

Die DE 103 39 329 A1 beschreibt ein durch ein Redoxinitiatorsystem härtendes Zweikomponenten-System mit steuerbarer Topfzeit, bestehend aus einem Emulsionspolymerisat oder mehreren Emulsionspolymerisaten und einem ethylenisch ungesättigten Monomer oder einer Monomermischung aus ethylenisch ungesättigten Monomeren, wobei sowohl das Emulsionspolymerisat als auch das Monomer oder die Monomermischung eine der Komponenten eines Redoxinitiatorsystems enthalten können. Die Steuerung der Topfzeit erreicht man durch Absorption wenigstens einer Komponente des Redoxinitiatorsystems am Polymeren. Dabei wird die niedermolekulare Initiatorkomponente physikalisch in Polymerteilchen verkapselt, welche durch Emulsionspolymerisation hergestellt werden. Kommt das verkapselte Polymer bei der Anwendung des Zweikomponenten-Systems mit Monomer zusammen quillt das Polymer, die ehemals verkapselte und/oder absorbierte Initiatorkomponente wird frei und kann ihre Wirkung entfalten. Obwohl diese "Verkapselung" einer Komponente des Initiatorsystems im Polymer bereits eine sehr günstige und variable Steuerung

der Topfzeit erlaubt, ist eine derartige Regelung doch noch in mancherlei Hinsicht verbesserungswürdig.
Hierbei handelt es sich einerseits um die Anwendungssicherheit. Durch Überlagerung kann beispielsweise die Konzentration der im Polymer verkapselten Komponente sinken, etwa durch Migration. In der Folge kann die Reaktivität des Systems gegebenenfalls von den Sollwerten abweichen.

Andererseits ist es bereits an sich schwierig, bei dem in der DE 103 39 329 A1 beschriebenen System eine hohe Beladung des Polymers mit der verkapselten Komponente zu erreichen. In der Praxis zeigen sich bei höheren Beladungen, z.B. 5 % oder darüber, Effekte, die auf nicht vollständigen Einschluß des Aktivators schließen lassen. Nun kann es jedoch sein, dass man besonders reaktive Systeme benötigt, so dass ein sehr hoher Beladungsgrad von teilweise bis zu 40 % (w/w) oder sogar darüber (> 40 % [w/w])erwünscht ist.

Schließlich muss auch bei einem hohen Beladungsgrad und gerade dann die Langzeit-Sicherheit des Beladungsgrades gewährleistet werden.

### 3. Aufgabe

In Anbetracht des eingangs genannten und diskutierten Standes der Technik war es Aufgabe der Erfindung, bei Raumtemperatur härtende Zwei- oder Mehrkomponenten-Systeme zur Verfügung zu stellen, deren Topfzeit in weiten Grenzen einstellbar ist und die trotzdem zu einem definierten Zeitpunkt ohne Energiezufuhr oder äußeren mechanischen Impuls schnell und vollständig aushärten.

Ferner bestand die Aufgabe darin, auch in dünnen Schichten ohne Luftausschluß eine vollständige Aushärtung zu erreichen.

Eine weitere erfindungsgemäß zu lösende Aufgabe besteht darin, geruchliche Belästigungen zu minimieren und bei der Anwendung die Konzentration an Monomer in der Luft unter den für das jeweilige Monomer gültigen Grenzwerten zu halten.

Eine weitere Aufgabe war es, einen großen Variationsbereich der Aktivatorkonzentration zu ermöglichen.

Weiterhin sollte die Topfzeit unabhängig von der Lagerdauer des Zwei- oder Mehrkomponentensystems gemacht werden. So werden Topfzeiten häufig durch eine bestimmte Konzentration an Inhibitoren eingestellt. Nach längerer Lagerung unter ungünstigen Bedingungen können die Inhibitoren teilweise verbraucht sein, so dass die Topfzeit kürzer ist als gewünscht.

Unter anderem war es auch Aufgabe der Erfindung ein System anzugeben, dass dem vorgenannten Spektrum an Eigenschaften in Summe genügen kann und dennoch einfach und sicher handhabbar ist.

Schließlich sollte die Erfindung auch die als Zwischenprodukte zur Bereitstellung des Systems notwendigen Polymerisate bereitstellen ebenso wie ein Verfahren zu deren Herstellung.

Auch die Angabe von Verwendungen für das erfindungsgemäße System war zu leisten.

### 4. Lösung

Die erfindungsgemäßen Aufgaben oder Teilaspekte der erfindungsgemäßen Aufgaben werden gelöst durch ein neues Emulsionspolymerisat erhältlich durch Polymerisation einer Mischung aufweisend

a) 5 bis 99,9 Gew.-% eines oder einer Mehrzahl von Monomeren mit einer Wasserlöslichkeit < 2 Gew.-% bei 20 °C ausgewählt aus der Gruppe bestehend aus monofunktionellen (Meth)acrylatmonomeren, Styrol und Vinylestern;
b) 0 bis 70 Gew.-% eines oder einer Mehrzahl mit den Monomeren a) copolymerisierbaren Monomeren;
c) 0 bis 20 Gew.-% einer oder einer Mehrzahl zweifach oder mehrfach vinylisch ungesättigter Verbindungen;
d) 0 bis 20 Gew.-% eines oder einer Mehrzahl polarer Monomere mit einer Wasserlöslichkeit > 2 Gew.-% bei 20°C; und
e) 0,1 - 95 Gew.-% wenigstens eines Aktivators,

wobei die Komponenten a) bis e) zusammen 100 Gew.-% der polymerisierbaren Bestandteile der Mischung ergeben, wobei sich das Emulsionspolymerisat dadurch auszeichnet, dass

e1) der Aktivator eine Verbindung der Formel I ist,

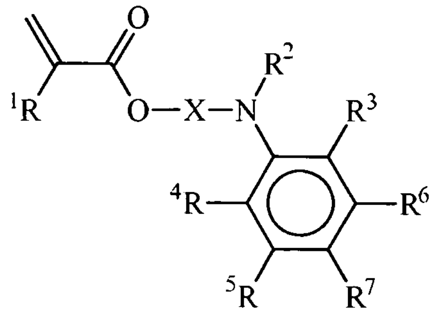

(I)

worin

- $R^1$ Wasserstoff oder Methyl ist;
- X eine lineare oder verzweigte Alkandiylgruppe mit 1 bis 18 Kohlenstoffatomen ist, die ein oder mehrfach mit Hydroxylgruppen und / oder mit $C_1$ - $C_4$ Alkoxygruppen substituiert sein kann;
- $R^2$ Wasserstoff oder einen linearen oder verzweigten Alkylrest mit 1 bis 12 Kohlenstoffatomen bedeutet, der gegebenenfalls ein oder mehrfach mit Hydroxylgruppen oder $C_1$ - $C_4$ -Alkoxygruppen substituiert ist, wobei die Hydroxylgruppen in $R^2$ partiell mit (Meth)acrylsäure verestert sein können.
- $R^3$, $R^4$, $R^5$, $R^6$ und $R^7$ unabhängig voneinander Wasserstoff oder eine lineare oder verzweigte Alkyl- oder Alkoxygruppe mit 1 bis 8 Kohlenstoffatomen bedeuten, die ein oder mehrfach mit Hydroxylgruppen substituiert sein können;

und wobei gegebenenfalls zwei der Reste $R^3$ bis $R^7$ miteinander zu einem fünfbis siebengliedrigen Ring verbunden sind und gegebenenfalls ein kondensiertes aromatisches Ringsystem mit dem Phenylrest bilden;
und dass

e2) der Aktivator e) kovalent an das Emulsionspolymerisat gebunden ist, und dass die Bestandteile a) bis e) als Kern-Schale-Polymerisate eingesetzt werden.

Ein derartiges Emulsionspolymerisat erlaubt als Zwischenprodukt die Schaffung von äußerst vorteilhaften durch ein Redoxinitiatorsystem härtenden Zwei- oder Mehrkomponenten-System mit steuerbarer Topfzeit.
Im Hinblick auf dieses System werden die der Erfindung zugrunde liegenden Aufgaben speziell gelöst durch ein Zwei- oder Mehrkomponenten-System umfassend

A) 0,8 - 69,94 Gew.-% eines erfindungsgemäßen Emulsionspolymerisats;
B) 30 - 99,14 Gew.-% eines oder einer Mehrzahl ethylenisch ungesättigter Monomere;
C) 0,05 -10 Gew.-% Peroxide; gegebenenfalls
D) 0 - 60 Gew.-% Oligomere oder Polymere;
E) 0,01 - 2 Gew.-% eines Polymerisationsinhibitors; und gegebenenfalls
F) 0 - 800 Gewichtsteile Hilfs- und Zusatzstoffe;

wobei die Summe der Bestandteile A) + B) + C) + D) +E) 100 Gew.-% ergibt und sich die Menge von F) auf 100 Gewichtsteile der Summe A) + B) + C) + D) + E) bezieht.
In der Regel liegen die Komponenten B), D), E) und F) als lagerfähige Mischung vor, während die Komponenten A) und C) dieser Mischung vor der Anwendung zugemischt werden.
Erfindungsgemäße Zwei- oder Mehrkomponentensysteme wiederum lassen sich mit großem Vorteil in Klebstoffen, Gießharzen, Bodenbeschichtungen, Massen für Reaktivdübel, Dentalmassen oder in Abdichtmassen einsetzen Mit den erfindungsgemäßen Zusammensetzungen lässt sich ein breiter Bereich der Konzentration des Aktivators (Variationsbereich) realisieren.
Ein besonderer Vorteil liegt darin, dass bei hohen Aktivatorkonzentrationen in Komponente A weniger von A vor der Anwendung zum Zwei- oder Mehrkomponentensystem zugemischt werden muss.
Auch die Möglichkeit zur Variation der Reaktivität ist vorteilhaft. Bei gleich bleibender Zusatzmenge der Komponente A kann die Reaktivität durch verschieden hohe Konzentrationen des Aktivators in A variiert werden.

## 5. Detaillierte Beschreibung der Erfindung

### Das Emulsionspolymerisat = Komponente A

Die Komponente A ist erhältlich durch Polymerisation einer Mischung aufweisend

a) 5 bis 99,9 Gew.-% eines oder einer Mehrzahl von Monomeren mit einer Wasserlöslichkeit < 2 Gew.-% bei 20 °C ausgewählt aus der Gruppe bestehend aus monofunktionellen (Meth)acrylatmonomeren, Styrol und Vinylestern;
b) 0 bis 70 Gew.-% eines oder einer Mehrzahl mit den Monomeren a) copolymerisierbaren Monomeren;
c) 0 bis 20 Gew.-% einer oder einer Mehrzahl zweifach oder mehrfach vinylisch ungesättigter Verbindungen;
d) 0 bis 20 Gew.-% eines oder einer Mehrzahl polarer Monomere mit einer Wasserlöslichkeit > 2 Gew.-% bei 20°C; und
e) 0,1 - 95 Gew.-% wenigstens eines Aktivators,

wobei die Bestandteile a) bis e) zusammen 100 Gew.-% der polymerisierbaren Bestandteile der Mischung ergeben, nach welcher das Emulsionspolymerisat = Komponente A resultiert,
wobei

e1) der Aktivator eine Verbindung der Formel I ist,

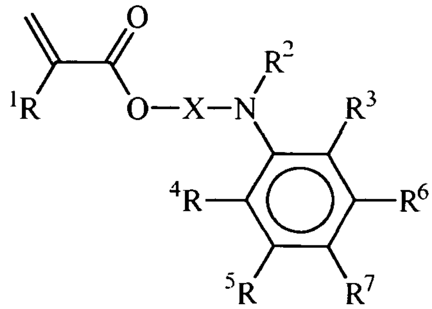

(I)

worin

- $R^1$ Wasserstoff oder Methyl ist;
- X eine lineare oder verzweigte Alkandiylgruppe mit 1 bis 18 Kohlenstoffatomen ist, die ein oder mehrfach mit Hydroxylgruppen und / oder mit $C_1$ - $C_4$ Alkoxygruppen substituiert sein kann;
- $R^2$ Wasserstoff oder einen linearen oder verzweigten Alkylrest mit 1 bis 12 Kohlenstoffatomen bedeutet, der gegebenenfalls ein oder mehrfach mit Hydroxylgruppen oder $C_1$ - $C_4$ -Alkoxygruppen substituiert ist
- $R^3$, $R^4$, $R^5$, $R^6$ und $R^7$ unabhängig voneinander Wasserstoff oder eine lineare oder verzweigte Alkyl- oder Alkoxygruppe mit 1 bis 8 Kohlenstoffatomen bedeuten, die ein oder mehrfach mit Hydroxylgruppen substituiert sein können, wobei die Hydroxylgruppen partiell mit (Meth)acrylsäure verestert sein können; und wobei gegebenenfalls zwei der Reste $R^3$ bis $R^7$ miteinander zu einem fünf- bis siebengliedrigen Ring verbunden sind und gegebenenfalls ein kondensiertes aromatisches Ringsystem mit dem Phenylrest bilden;

wobei

e2) der Aktivator e) kovalent in das Emulsionspolymerisat eingebaut ist, und wobei die Bestandteile a) bis e) als Kern-Schale-Polymerisate eingesetzt werden.

Die Schreibweise (Meth)acrylat bedeutet hier sowie im gesamten Kontext der Erfindung sowohl Methacrylat, wie z.B. Methylmethacrylat, Ethylmethacrylat usw., als auch Acrylat, wie z.B. Methylacrylat, Ethylacrylat usw., sowie Mischungen aus beiden.
Das Emulsionspolymerisat = Komponente A) ist vorzugsweise im Wesentlichen aus (Meth)acrylatmonomeren sowie Styrol und/oder Styrolderivaten und/oder Vinylestern aufgebaut.
Besonders bevorzugt ist der Aufbau aus mindestens 80 % Methacrylat- und Acrylatmonomeren, ganz besonders bevorzugt ist der Aufbau aus ausschließlich Methacrylat- und Acrylatmonomeren.

Komponente A a)

Beispiele für monofunktionelle Methacrylat- und Acrylatmonomere mit einer Wasserlöslichkeit < 2 Gew.-% bei 20 °C sind Methyl(meth)acrylat, Ethyl(meth)acrylat, Propyl(meth)acrylat, Isopropyl(meth)acrylat, n-Butyl(meth)acrylat, Isobutyl(meth)acrylat, tert.-Butyl(meth)acrylat, Hexyl(meth)acrylat, Ethylhexyl(meth)acrylat, Isodecylmethacrylat, Laurylmethacrylat, Cyclohexyl(meth)acrylat, Tetrahydrofurfuryl(meth)acrylat, Isobornyl(meth)acrylat, Benzyl(meth)acrylat, Phenyl(meth)acrylat, Phenylethyl(meth)acrylat, 3,3,5-Trimethylcyclohexyl(meth)acrylat. Methoden zur Bestimmung der Wasserlöslichkeit von organischen Verbindungen sind dem Fachmann geläufig.

Unter Styrolderivaten versteht man beispielsweise $\alpha$-Methylstyrol, Chlorstyrol oder p-Methylstyrol. Beispiele für Vinylester sind Vinylacetat und längerkettige Derivate wie Vinylversatat.

Bevorzugt werden Methacrylatmonomere, insbesondere Methylmethacrylat, eingebaut, um eine hohe Glastemperatur zu erreichen und Methacrylate mit einer C-Zahl > 4 in der Seitenkette und Acrylate, um die Glastemperatur abzusenken. Vorteilhafterweise werden die Monomeren so kombiniert, dass eine Glastemperatur über 60°C resultiert, bevorzugt über 80° C und insbesondere über 100° C, wenn das Emulsionspolymerisat A durch Trocknung isoliert werden soll. Die Glastemperaturen werden gemessen nach EN ISO 11357. Soll das Emulsionspolymerisat A dem Zwei- oder Mehrkomponenten-System als wäßrige Dispersion zugesetzt werden, so kann die Glastemperatur niedriger liegen. Um den Monomeren B einen ausreichend hohen Anquellwiderstand entgegenzusetzen ist meist eine Glastemperatur oberhalb Raumtemperatur vorteilhaft. Bevorzugt liegt sie über 30 °C, besonders bevorzugt über 40 °C, insbesondere über 60 °C.

Damit ist nicht ausgeschlossen, dass in bestimmten Fällen Glastemperaturen unterhalb Raumtemperatur vorteilhaft sind. Das kann beispielsweise der Fall sein, wenn die Lösekraft der für Komponente B verwendeten Monomeren niedrig ist, so dass die Anquellung zu lange dauert.

Bei bekannter Glastemperatur von Homopolymeren lassen sich die Glastemperaturen der Copolymeren nach folgender Formel von Fox in erster Näherung berechnen:

$$\frac{1}{T_g} = \frac{w_A}{T_{gA}} + \frac{w_B}{T_{gB}} + \frac{w_C}{T_{gC}} + \dots$$

Dabei bedeuten: $T_g$ die Glastemperatur des Copolymerisats (in K), $T_{gA}$, $T_{gB}$, $T_{gC}$ usw. die Glastemperaturen der Homopolymerisate der Monomere A, B, C usw. (in K). $w_A$, $w_B$, $w_C$ usw. stellen die Massenanteile der Monomere A, B, C, usw. im Polymer dar.

Je höher die Glastemperatur des Polymeren ist, desto größer ist der Anquellwiderstand gegenüber den vor der Anwendung zugesetzten Monomeren und somit die Topfzeit. Ebenso bewirkt eine steigende Molmasse eine Erhöhung des Anquellwiderstands.

Insofern sind besonders bevorzugte Polymerisate dadurch gekennzeichnet, dass a) aus ein oder mehreren Methacrylatmonomeren und/oder Acrylatmonomeren besteht. Ganz besonders zweckmäßig ist a) Methylmethacrylat.

Komponente A b)

Beispiele für die Komponente A b) sind Maleinsäureanhydrid, Itakonsäureanhydrid sowie Ester der Itakon- und Maleinsäure. Ihr Anteil am Emulsionspolymerisat kann bis zu 70 Gew.-% betragen, vorzugsweise sind von 0-30 Gew.-% , insbesondere 0-10 Gew.-% enthalten. Ganz besonders bevorzugt wird auf Komponente A b) verzichtet.

Komponente A c)

Der Einbau höherer Anteile an zweifach und/oder mehrfach ungesättigten Monomeren (Vernetzer) beschränkt den erreichbaren Quellungsgrad in der Formulierung und kann auf nanoskaliger Ebene zu einem inhomogen Polymerisat führen. Dies muss nicht in jedem Fall nachteilig sein, wird aber bevorzugt nicht angestrebt. Deshalb wird der Gehalt an mehrfach ungesättigten Monomeren auf vorzugsweise auf 20 Gew.-%, bezogen auf Komponente A, beschränkt, noch mehr bevorzugt liegt er unter 10 Gew.-%, besonders bevorzugt unter 2 Gew.-%, insbesondere unter 0,5 Gew.-% oder es wird ganz auf mehrfach ungesättigte Monomere verzichtet.

Zu im Rahmen der Erfindung mit Erfolg einsetzbaren mehrfach ungesättigten Monomeren (Vernetzern) gehören unter anderem Ethylenglykoldi(meth)acrylat sowie Diethylenglykoldi(meth)acrylat, Triethylenglykoldi(meth)acrylat und deren höheren Homologe, 1,3- und 1,4-Butandioldi(meth)acrylat, 1,6-Hexandioldi(meth)acrylat, Trimethylolpropandi (meth)acrylat oder (Meth)acrylate von ethoxiliertem Trimethylolpropan, Triallylcyanurat und/oder Allyl(meth)acrylat.

Komponente A d)

Der Anquellwiderstand kann auch durch den Einbau polarer Monomere, wie Methacrylamid oder Methacrylsäure in das Emulsionspolymerisat gesteuert werden. Dieser steigt mit steigender Menge Methacrylamid bzw. Methacrylsäure an.

Beispiele für weitere polare Monomere sind z.B. Acrylsäure, Acrylamid, Acrylnitril, Methacrylnitril, Itakonsäure, Maleinsäure oder N-Methacryloyloxyethylethylenharnstoff und N-Methacryloylamidoethylethylenharnstoff. Auch N-Methylolacrylamid oder - methacrylamid und deren Ether sind denkbar, sofern ihr Anteil so beschränkt wird, dass trotz Vernetzung der Dispersionsteilchen deren Anquellung hinreichend möglich ist und die Auslösung der Polymerisation nicht beeinträchtigt wird.

Der Anteil an N-Methylolacrylamid oder-methacrylamid sollte 10 Gew. %, bezogen auf Komponente A, möglichst nicht überschreiten. Bevorzugt ist ein Gehalt unter 5 Gew.-%, besonders bevorzugt unter 2 Gew.-%, insbesondere 0 Gew.-%.

Weitere polare Monomere sind Hydroxyethyl(meth)acrylat, Hydroxypropyl(meth)acrylat, Homologe des Alkoxypolyethylenglykolmethacrylat, des Alkoxypolypropylenglykolmethacrylat, des Methacryloyloxypolyethylen- und - polypropylenglykol sowie von Vinyloxypolyethylen- und polypropylenglykol. Alle genannten Monomeren können auch als Mischform von Ethylen- und Propylenglykolwiederholungseinheiten vorliegen. Der Polymerisationsgrad kann 2 bis 150 betragen, bevorzugt 2 bis 25. Alkoxy- steht in erster Linie für Methyl-, Ethyl- und Butylreste. Längere Alkylketten, wie z.B. C18, sind auch möglich, aber nicht bevorzugt. Besonders bevorzugt ist ein Methylrest.

Der Anteil der polaren Monomeren hängt in erster Linie von der angestrebten Topfzeit der Formulierung ab, er ist aber auch mit der Glastemperatur des Polymeren verknüpft. Je niedriger die Glastemperatur liegt, desto höher ist der benötigte Anteil an polaren Monomeren, um einen bestimmten Anquellwiderstand zu erreichen. Weiterhin ist der Anteil an polaren Monomeren auf die Lösekraft der in der Formulierung eingesetzten Monomeren B abzustimmen.

In der Regel liegt der Anteil an polaren Monomeren im Bereich von 0 Gew.-% und 20 Gew.-%, bevorzugt von 1 Gew.-% bis 10 Gew.-%, besonders bevorzugt von 2 Gew.-% bis 5 Gew.-%, insbesondere von 3 Gew.-% bis 5 Gew.-%, bezogen auf Komponente A. Sind kurze Topfzeiten gewünscht, beispielsweise wenige Minuten, oder ist die Lösekraft der Monomeren in Komponente B gering, so kann es vorteilhaft sein, den Gehalt auf unter 2 Gew.-% zu beschränken oder ganz auf polare Monomere zu verzichten.

Methacrylamid und Acrylamid sowie Methacrylsäure und Acrylsäure sind besonders wirksam und werden deshalb bevorzugt, wenn lange Topfzeiten angestrebt werden. Besonders bevorzugt ist eine Kombination aus Methacrylamid oder Acrylamid mit Methacrylsäure oder Acrylsäure in den Gewichtsverhältnissen von 3 zu 1 bis 1 zu 3.

Komponente A e)

Die im Rahmen der Erfindung mit Erfolg einzusetzende Komponente Ae) gehorcht der oben bezeichneten allgemeinen Formel I.

Für die Offenbarung der Erfindung versteht man unter einer linearen oder verzweigten Alkandiylgruppe mit 1 bis 18 Kohlenstoffatomen, einen unverzweigten oder verzweigten Kohlenwasserstoffrest mit 1 bis 18 Kohlenstoffatomen, wie z.B. den Methandiyl-(=Methylengruppe), Ethandiyl-, Propandiyl-, 1-Methylethandiyl-, 2-Methylpropandiyl-, 1,1-Dimethylethandiyl-, Pentandiyl-, 2-Methylbutandiyl-, 1,1-Dimethylpropandiyl-, Hexandiyl-, Heptandiyl-, Octandiyl-, 1,1,3,3-Tetramethylbutandiyl-, Nonandiyl-, Isononandiyl-, Decandiyl-, Undecandiyl-, Dodecandiyl- oder Hexadecandiylrest.

Unter dem Begriff linearen oder verzweigten Alkylrest mit 1 bis 8 Kohlenstoffatomen versteht man für die Erfindung Reste wie z. B. den Methyl-, Ethyl-, Propyl-, 1-Methylethyl-, 2-Methylpropyl, 1,1-Dimethylethylrest, Pentyl-, 2-Methylbutyl-, 1,1-Dimethylpropyl-, Hexyl-, Heptyl-, Octyl-, oder den 1,1,3,3-Tetramethylbutylrest.

Unter dem Begriff linearen oder verzweigten Alkylrest mit 1 bis 12 Kohlenstoffatomen versteht man für die Erfindung Reste mit 1 bis 8 Kohlenstoffatomen wie zuvor beschrieben sowie z.B. den Nonyl-, Isononyl-, Decyl-, Undecy- oder den Dodecylrest.

Unter dem Begriff $C_1$-$C_4$-Alkoxygruppen werden für die Erfindung Alkoxygruppen verstanden, bei denen der Kohlenwasserstoffrest ein verzweigter oder unverzweigter Kohlenwasserstoffrest mit 1 bis 4 Kohlenstoffatomen ist, wie z. B. der Methyl-, Ethyl-, Propyl-, 1-Methylethyl-, 2-Methylpropyl oder 1,1-Dimethylethylrest.

Unter dem Begriff lineare oder verzweigte Alkoxygruppe mit 1 bis 8 Kohlenstoffatomen werden für die Erfindung Alkoxygruppen verstanden, bei denen der Kohlenwasserstoffrest ein verzweigter oder unverzweigter Kohlenwasserstoffrest mit 1 bis 8 Kohlenstoffatomen ist, wie z. B. der Methyl-, Ethyl-, Propyl-, 1-Methylethyl-, 2-Methylpropyl, 1,1-Dimethylethylrest, Pentyl-, 2-Methylbutyl-, 1,1-Dimethylpropyl-, Hexyl-, Heptyl-, Octyl-, oder der 1,1,3,3-Tetramethylbutylrest.

Wie die Formel (I) zeigt handelt es sich bei den möglichen Aktivatorkomponenten Ae) allgemein um (meth) acryloyl-funktionalisierte Aminabkömmlinge. Dabei geht man generell bei den Aktivator- oder Beschleunigerkomponenten von modifizierten Aminen, wie 2-N-(Ethylanilino)ethanol oder 2-N-(Ethylanilino)propanol, aus und setzt diese Amine

zu polymerisierbaren Beschleuniger-/Aktivatorkomponenten um, vorzugsweise durch Einführung von (Meth)acrylatgruppen. Entsprechend können beispielsweise auch m-Toluidin- sowie Xylidinderivate oder weitere Derivate als Ausgangspunkt zum Erhalt der Beschleunigerkomponente eingesetzt werden.

Zu bevorzugt einsetzbaren Aktivator- / Beschleunigerkomponenten Ae) gehören unter anderem folgende Verbindungsklassen: N-((Meth)acryloyl(poly)oxyalkyl)-N-alkyl-(o,m,p)-(mono,di,tri,tetra,penta)alkylanilin, N-((Meth)acryloyl (poly)oxyalkyl)-N-(arylalkyl)-(o,m,p)-(mono,di,tri,tetra,penta)alkylanilin, N-((Meth)acryloyl(poly)oxyalkyl)-N-alkyl-(o,m, p)-(mono,di,tri,tetra,penta)alkylnaphthylamin. Bevorzugt werden .N-((Meth)acryloyloxyethyl)-N-methylanilin, N-((Meth) acryloyloxypropyl)-N-methylanilin, N-((Meth)acryloyloxypropyl)-N-methyl-(o,m,p)-toluidin, N-((Meth)acryloyloxyethyl)-N-methyl-(o,m,p)-toluidin, N-((Meth)acryloylpolyoxyethyl)-N-methyl-(o,m,p)-toluidin. Diese Stoffe werden einzeln oder in Mischung von zwei oder mehreren eingesetzt.

Besonders zweckmäßige Emulsionspolymerisate für die Zwecke der Erfindung sind methacryloylfunktionalisierte Substanzen, d.h. solche Verbindungen der Formel (I) worin $R^1$ Methyl ist.

In einer weiteren bevorzugten Ausführungsform kennzeichnen sich die Polymersiate dadurch, dass in der Formel (I) X eine Ethandiyl-, also eine Ethylengruppe $-CH_2-CH_2-$ ist.

In einer anderen besonders bevorzugten Ausführungsform ist das Emulsionspolymerisat dadurch gekennzeichnet, dass X in der Formel (I) eine hydroxylsubstituierte Propandiylgruppe, nämlich eine 2-Hydroxypropylengruppe $-CH_2-CH(OH)-CH_2-$ ist.

Weitere bevorzugte Aktivatoren ergeben sich dadurch, dass der Rest $R^2$ in der Formel (I) ausgewählt ist aus der Gruppe bestehend aus Methyl, Ethyl und 2-Hydroxyethyl.

Bevorzugt enthält e1) nur eine (Meth)acryloylgruppe. Möglich, wenn auch nicht bevorzugt, ist eine mehrfache Unsättigung durch partielle Veresterung der Hydroxylgruppen in $R^2$ mit (Meth)acrylsäure, die bei der Synthese nicht immer ganz vermieden werden kann. Ein Gehalt an solchen vernetzend wirkenden Strukturen ist unkritisch, solange er die Verwendbarkeit der Emulsionspolymerisate A) in den Zwei- oder Mehrkomponentensystemen nicht beeinträchtigt, beispielsweise durch nicht mehr ausreichende Quellbarkeit des Emulsionspolymerisats in Komponente B) aufgrund eines zu hohen Vernetzungsgrades. Typischerweise ist ein Anteil an mehrfach ungesättigtem Aktivatormonomer unter 5 Gew.-% bezogen auf die Polymerzusammensetzung nicht unbedingt prohibitiv, bevorzugt sind unter 3, insbesondere unter 1 Gew.-%. Höhere Gehalte sind allerdings nicht ausgeschlossen. Der Fachmann kann leicht bestimmen, ob das Monomer geeignet ist, indem er beispielsweise prüft, ob ein damit hergestelltes Emulsionspolymerisat A) im Zwei- oder Mehrkomponentensystem die Polymerisation im gewünschten Zeitintervall auslöst und ob die Polymerisation schnell und vollständig abläuft und das Polymerisat die gewünschten Eigenschaften besitzt.

Ebenfalls bevorzugt sich solche Polymerisate als Aktivatoren, bei denen einer der Reste $R^3$ bis $R^7$ Methyl ist während die verbleibenden vier Reste Wasserstoff sind.

Außerdem sind solche Polymerisate zweckmäßig, die dadurch gekennzeichnet sind, dass in der Formel (I) zwei der Reste $R^3$ bis $R^7$ Methyl sind während die verbleibenden drei Reste Wasserstoff sind.

Der Anteil des polymerisierbaren Aktivators A e) in Komponente A kann zwischen 0,1 Gew.-% und 95 Gew.-% betragen. Vorzugsweise wird er möglichst hoch gewählt, beispielsweise zwischen 5 Gew.-% und 60 Gew.-%, besonders bevorzugt 10 Gew.-% - 60 Gew.-%, insbesondere 20 Gew.-% - 50 Gew.-%. Die obere Grenze wird durch das Verhalten des ausgewählten Aktivators bei der Emulsionspolymerisation bestimmt. Der Fachmann wird darauf achten, dass durch einen zu hohen Anteil weder nicht akzeptable Mengen Koagulat gebildet werden, noch zu hohe Restmonomergehalte verbleiben. Es kann auch sein, dass die spezifische Wirksamkeit des Aktivators mit steigender Einbaumenge abnimmt. Da der polymerisierbare Aktivator eine eher teure Monomerkomponente darstellt, wird der Fachmann bestrebt sein, ein Kompromiß zwischen möglichst hoher Einbaumenge und Wirtschaftlichkeit zu finden.

Das Emulsionspolymerisat kann auch als Kern-Schale-Polymer aufgebaut sein. Kern-Schale-Polymer steht hier für ein Polymerisat, das durch eine zwei- oder mehrstufige Emulsionspolymerisation hergestellt wurde, ohne dass der Kern-Schale-Aufbau beispielsweise elektronenmikroskopisch gezeigt wurde. Wird der polymerisierbare Aktivator nur im Kern, also in der ersten Stufe, eingebaut, so trägt ein solcher Aufbau dazu bei, dass der Aktivator bis zur Anquellung nicht für das Peroxid zugänglich ist und somit eine vorzeitige Polymerisation verhindert wird. Eine Ausführungsform ist, dass die polaren Monomeren auf die Schale beschränkt werden, Kern und Schale, abgesehen vom polymerisierbaren Aktivator im Kern, aber ansonsten gleich aufgebaut sind. In einer anderen Ausführungsform können Kern und Schale sich in der Monomerzusammensetzung wesentlich unterscheiden, was sich beispielsweise auf die jeweilige Glastemperatur auswirkt. In diesem Fall ist es vorteilhaft, wenn die Glastemperatur der Schale oberhalb der des Kerns liegt, vorzugsweise oberhalb 60 °C, besonders bevorzugt oberhalb von 80°C, insbesondere oberhalb von 100 °C. Zusätzlich können auch in dieser Ausführungsform die polaren Monomeren auf die Schale beschränkt sein. Im Allgemeinen wird der Fachmann nur dann den komplexeren Kern-Schale-Aufbau wählen, wenn er damit vorteilhafte Eigenschaften bewirken kann. Der bessere Schutz des Aktivators gegen vorzeitigen Kontakt mit dem Peroxid durch eine Schale kann das Ziel sein. Das Aktivatormonomer wird dann vorzugsweise in den Kern eingebaut. Ebenso kann es darum gehen, die ausgehärteten Polymerisate flexibler zu machen. In solchen Fällen wird der Kern mit relativ niedriger Glastemperatur eingestellt. Die Schale mit höherer Glastemperatur hat dann die Aufgabe, den gewünschten Anquellwiderstand und ggf.

die Isolierung als Feststoff zu gewährleisten. Das Gewichtsverhältnis von Kern zu Schale hängt davon ab, wie gut der Aktivator geschützt werden soll oder welche Effekte durch diesen Aufbau erwartet werden. Im Prinzip kann es zwischen1 : 99 und 99 : 1 liegen, d. h. es ist in der Regel unkritisch, solange der Zweck des Emulsionspolymersats A, die Polymerisation des Zwei- oder Mehrkomponentensystems in der gewünschten Weise zu aktivieren, nicht beeinträchtigt wird.

Ist beabsichtigt durch die Schale den Aktivator zu schützen, so wird man den Schalenanteil in der Regel auf das notwendige Maß beschränken, um einen hohen Aktivatoranteil im Emulsionspolymerisat zu ermöglichen.
Sollen durch den Aufbau besondere Effekte, z.B. eine Flexibilisierung der ausgehärteten Polymersysteme durch ein Kern-Polymer mit niedriger Glastemperatur, erreicht werden, so ist das Kern-Schale-Verhältnis auf die gewünschten Effekte abzustimmen. Meist wird der Fachmann den Schalenanteil zwischen 10 Gew.-% und 50 Gew.-% einstellen, vorzugsweise zwischen 20 Gew.-% und 40 Gew.-%, insbesondere zwischen 25 Gew.-% und 35 Gew.-%.

Insofern betrifft die Erfindung auch ein Verfahren zur Herstellung eines erfindungsgemäßen Emulsionspolymerisats, bei welchem man die Bestandteile a) bis e) der Komponente A) in wäßriger Emulsion polymerisiert.

Die Emulsionspolymerisation wird dabei in einer dem Fachmann im Allgemeinen bekannten Art und Weise durchgeführt. Die Durchführung einer Emulsionspolymersiation ist beispielhaft in EP 0376096 B1 beschrieben.

Vorzugsweise wird ein Initiator gewählt, der mit dem polymerisierbaren Aktivator A e) kein Redoxsystem bildet. Geeignet sind beispielsweise Azoinitiatoren wie das Na-Salz der 4,4'-Azobis-(4-cyanovaleriansäure).

Der Feststoff der Komponente A kann durch bekannte Verfahren aus der Dispersion gewonnen werden. Hierzu zählen Sprühtrocknung, Gefrierkoagulation mit Abnutschen und Trocknen sowie das Abpressen mittels Extruder. Bevorzugt wird das Polymerisat durch Sprühtrocknung gewonnen.
Wenn eine gewisse Menge Wasser in der Anwendung nicht stört, kann Komponente A auch als wäßrige Dispersion dem System zugesetzt werden.

Die Molmasse der Komponente A) ausgedrückt als Gewichtsmittel des Molekulargewichts $M_W$ beeinflusst in gewissem Maße den Anquellwiderstand. Hohe Gewichtsmittel des Molekulargewichts $M_W$ wirken tendenziell erhöhend auf den Anquellwiderstand, während niedrigere Gewichtsmittel des Molekulargewichts $M_W$ senkend wirken. Damit ist die erwünschte Topfzeit unter anderem dafür maßgebend, ob der Fachmann eine hohe Molmasse wählt oder eine eher niedrige.

Wenn über die Molmasse keine besonderen Effekte erzielt werden sollen, so wird der Fachmann die Molmasse in der Regel zwischen 10.000 g/mol und 5.000.000 g/mol, bevorzugt zwischen 50.000 g/mol und 1.000.000 g/mol und ganz besonders bevorzugt zwischen 100.000 g/mol und 500.000 g/mol einstellen. Die Molmasse wird mittels Gelpermeationschromatographie bestimmt. Die Messung erfolgt in THF, als Eichstandard dient PMMA.

Der Anquellwiderstand kann auch durch die Wahl der Teilchengröße eingestellt werden. Je größer der Teilchendurchmesser desto geringer ist die Anquellgeschwindigkeit.

Die Primärpartikelgröße der Komponente A beträgt in der Regel zwischen 50 nm und 2 Mikrometer, bevorzugt zwischen 100 nm und 600 nm und ganz besonders bevorzugt zwischen 150 nm und 400 nm. Die Teilchengröße wird mit einem Mastersizer 2000 Version 4.00 gemessen.
Führt man das Verfahren zur Herstellung erfindungsgemäßer Polymerisate nach Art eines Kern-/Schalepolymerisationsverfahrens durch, ist es für die Erfindung in anbetracht der weiter oben gemachten Ausführungen besonders zweckmäßig, wenn man die Bestandteile a) bis e) in einer ersten Stufe als Kern polymerisiert und daran anschließend in wenigstens in einer weiteren Stufe als Schale eine Mischung der Bestandteile a) bis d). Hierdurch wird eine besonders gute Verkapselung oder Maskierung der Aktivatorkomponente erreicht.

In einer besonders bevorzugten Abwandlung des Verfahrens der Erfindung geht man so vor, dass man die Bestandteile a) bis e) für den Kern und die Bestandteile a) bis d) für die Schale so wählt, dass im resultierenden Polymerisat die Glastemperatur $T_{GS}$ mindestens einer Schale größer als die Glasemperatur $T_{GK}$ des Kerns ist, wobei die Glastemperaturen $T_G$ nach EN ISO 11357 bestimmt werden.

Noch eine weitere Verfahrensmodifikation sieht vor, dass man die Bestandteile a) bis d) für die Schale so wählt, dass im resultierenden Polymerisat die Glastemperatur $T_{GS}$ mindestens einer Schale größer als 80, bevorzugt größer als 100 °C ist, wobei die Glastemperatur $T_{GS}$ nach EN ISO 11357 bestimmt wird.

Die Emulsionspolymerisation ist grundsätzlich als Batch- oder Zulaufpolymerisation möglich, Zulaufpolymerisation ist bevorzugt. Ebenso ist die Herstellung von A) über eine Miniemulsionspolymerisation möglich. Die Vorgehensweisen sind dem Fachmann bekannt.

Vor der Anwendung wird das vorzugsweise sprühgetrocknete Emulsionspolymerisat A sowie die Komponente C in einem die Komponenten D, E und F enthaltenden Monomer oder einer Monomermischung suspendiert. Das suspendierte Polymer wird innerhalb eines bestimmten Zeitraums durch das Monomer oder die Monomere B angequollen. Damit wird die polymerfixierte Aktivatorkomponente für das Peroxid zugänglich und somit die Polymerisationsreaktion gestartet.

Aus den langen Topfzeiten nach der Mischung der Komponenten ist zu schließen, dass der polymerfixierte Aktivator hinreichend im Polymerteilchen verborgen ist. Überraschend ist der schnelle und starke Temperaturanstieg zu einem bestimmten Zeitpunkt, der zeigt, dass mit dem erfindungsgemäßen Verfahren eine lange Topfzeit eingestellt

werden kann, ohne dass die spätere Polymerisation beeinträchtigt wird.

**Komponente B: Die Monomeren**

Die Topfzeit der Formulierung aus den Komponenten A, B, C, D, E und F) kann durch die Anquellkraft der eingesetzten Monomeren in Komponente B beeinflusst werden. Während Methyl(meth)acrylat eine hohe Anquellkraft besitzt und damit zu niedrigeren Topfzeiten führt, erhöhen stärker hydrophobe Monomere, wie beispielsweise 1,4-Butandioldi(meth)acrylat und Monomere mit hohem Molekulargewicht, wie beispielsweise Ethyltrigykol(meth)acrylat in der Regel die Topfzeit.

Als Monomere können grundsätzlich alle Methacrylat- und Acrylatmonomeren und Styrol sowie deren Mischungen eingesetzt werden. Untergeordnete Anteile an anderen Monomeren wie Vinylacetat, Vinylversatat, Vinyloxypolyethylenglykol, Malein- und Fumarsäure und deren Anhydride oder Ester sind möglich, solange die Copolymerisation nicht gestört wird, sind aber nicht bevorzugt. Kriterien für die Auswahl der Monomeren sind deren Lösekraft, Polymerisationsschrumpf, Haftung auf dem Substrat, Dampfdruck, toxikologische Eigenschaften und Geruch. Beispiele für (Meth) acrylate sind Methyl(meth)acrylat, Ethyl(meth)acrylat, Propyl(meth)acrylat, Isopropyl(meth)acrylat, Butyl(meth)acrylat, Isobutyl(meth)acrylat, Hexyl(meth)acrylat, Ethylhexyl(meth)acrylat, Cyclohexyl(meth)acrylat, Tetrahydrofurfuryl(meth) acrylat, Isobornyl(meth)acrylat, Benzyl(meth)acrylat, Phenyl(meth)acrylat, Phenylethyl(meth)acrylat), 3,3,5-Trimethylcyclohexyl(meth)acrylat, Hydroxethyl(meth)acrylat, Hydroxypropyl(meth)acrylat, Methyl- oder Ethyltriglykolmethacrylat, Butyldiglykolmethacrylat, Ethylenglykoldi(meth)acrylat sowie Diethylenglykoldi(meth)acrylat, Triethylenglykoldi(meth) acrylat und deren höheren Homologe, Dipropylenglykoldi(meth)acrylat, Tripropylenglykoldi(meth)acrylat und deren höheren Homologe,1,3- und 1,4-Butandioldi(meth)acrylat, 1,6-Hexandioldi(meth)acrylat, 1,12-Dodecandioldi(meth)acrylat, Glycerindi(meth)acrylat, Trimethylolpropantri(meth)acrylat, Trimethylolpropandi(meth)acrylat, Tri(meth)acrylat eines ethoxilierten Trimethylolpropan mit 3-10 Mol Ethylenoxid, Di(meth)acrylat eines ethoxilierten Bisphenol-A mit 2-20 Mol Ethylenoxid, bevorzugt 2-10 Mol Ethylenoxid und / oder einem Polyethylenglykoldimethacrylat mit 1 - 15 Ethylenoxid-Einheiten und Allyl(meth)acrylat. Weitere Beispiele sind (Meth)acrylsäure, (Meth)acrylamid, N-Methylol(meth)acrylamid, Monoester der Malein- und Bernsteinsäure mit Hydroxyethylmethacrylat und der Phosphorsäureester von Hydroxyethyl (meth)acrylat, deren Anteil meist untergeordnet ist.

Unter anderem bevorzugt ist für die Komponente B) eine oder eine Mehrzahl von Verbindungen ausgewählt aus der Gruppe bestehend aus Ethyltriglykolmethacrylat, Tetrahydrofururylmethacrylat, Benzylmethacrylat, Isobornylmethacrylat, 1,4-Butandioldimethacrylat, Hydroxypropylmethacrylat, Trimethylolpropantrimethacrylat, Trimethacrylat eines ethoxilierten Trimethylolpropan mit 3 -10 Mol Ethylenoxid, Dimethacrylat eines ethoxilierten Bisphenol-A mit 2 - 10 Mol Ethylenoxid und / oder einem Polyethylenglykoldimethacrylat mit 1 -10 Ethylenoxid-Einheiten.

Besonders bevorzugt sind (Meth)acrylate mit einem Molekulargewicht oberhalb 140 g/mol, besonders bevorzugt oberhalb 165 g/mol und insbesondere oberhalb 200 g/mol.

Methacrylate erhalten gegenüber Acrylaten auch aus toxikologischen Gründen den Vorzug.

Neben langen Topfzeiten aufgrund geringer Anquellgeschwindigkeit haben Monomere mit hohem Molekulargewicht noch den Vorteil geringer Emissionen. Andererseits steigt ihre Viskosität mit der Molmasse in der Regel an und die Lösekraft für das Emulsionspolymerisat sinkt, so dass, insbesondere wenn Polymere oder Oligomere in nennenswerten Anteilen mitverwendet werden, ein Kompromiß eingegangen werden muß.

**Komponente C:**

Das Peroxid ist der Partner des Aktivators im Redoxsystem. Sein Anteil beträgt in der Regel zwischen 0,05 Gew.-% und 10 Gew.-%, bevorzugt 0,1 Gew.-% bis 5 Gew.-%. Meist wird ein Anteil von 0,5 Gew.-% - 5 Gew.-% gewählt, bevorzugt 0,5 Gew.-% - 3 Gew.-%, insbesondere 0,5 Gew.-% - 2 Gew.-%. Maßgebend für den Peroxidanteil ist, dass bei der vorgesehenen Anwendung eine vollständige Aushärtung in der gewünschten Zeit erfolgt und das ausgehärtete System die auf den Einsatzzweck abgestimmten Eigenschaften aufweist.

Das Peroxid liegt in der Regel phlegmatisiert z.B. in Weichmacher oder Wasser oder einem anderen Medium vor. Typische Peroxidgehalte dieser Peroxidformulieung liegen bei 20 Gew.-% - 60 Gew.-%. Als Peroxide kommen in erster Linie beispielsweise Dibenzoylperoxid und Dilaurylperoxid in Betracht.

Eine weitere Variante besteht darin, das Peroxid in einem Emulsionspolymerisat zu absorbieren (Komponente C'). In einer weiteren Ausführungsform der Erfindung besteht Komponente C also aus einem Emulsionspolymerisat enthaltend ein Peroxid (Komponente C'). Das Emulsionspolymerisat der Komponente C' kann gleich oder verschieden aufgebaut sein wie Komponente A, enthält aber keinen polymerisierbaren Aktivator als Comonomer. Typische Peroxidgehalte in Komponente C' liegen unter 20 Gew.-%, insbesondere unter 10 Gew.-%.

Nach Mischung aller Komponenten startet die Polymerisation erst dann, wenn die Polymerteilchen beider Komponenten A und C' angequollen sind.

Dabei ist es in der Regel unkritisch, ob die Emulsionspolymerisate A und C' gleich oder unterschiedlich zusammengesetzt sind, solange sich eine eventuelle Unverträglichkeit nicht nachteilig auswirkt.

**Komponente D:**

Als Oligomere können ungesättigte Polyester, sowie Polyurethan-(Meth)acrylate basierend auf Polyether-, Polyester-, oder Polycarbonat-diolen, sowie Mischungen derselben eingesetzt werden. Ferner können vinylterminierte Präpolymere auf Basis von Acrylnitril und Butadien eingesetzt werden. Ferner können Epoxid(meth)acrylate und auch sternförmige Copolymerisate eingesetzt werden, wie sie beispielsweise durch Polymerisation von (Meth)acrylaten in Gegenwart von mehrfunktionellen Mercaptanen zugänglich sind.
Vorzugsweise sind die Oligomeren mehrfach ungesättigt.
Es können ferner auch Polymere auf Basis von Polyacrylaten, Polyestern, Polyethern, Polycarbonaten oder den entsprechenden Copolymeren eingesetzt werden. Diese können sowohl gesättigt als auch ungesättigt sein. Das Mischungsverhältnis sowie die Einsatzmenge ist von der angestrebten Anwendung abhängig. Die Polymere bzw. ihr Anteil werden in der Regel so ausgewählt, daß die Viskosität der Mischung nicht negativ beeinflußt wird.
Die Molmasse der ungesättigten Oligomeren beträgt typischerweise 500 bis 20.000, insbesondere 1.000 bis 5.000 g/mol. Gesättigte Polymere haben typischerweise Molmassen oberhalb 20.000, beispielsweise 50.000 - 200.000 g/Mol. Es handelt sich in allen Fällen um Gewichtsmittelwerte des Molekulargewichts.

**Komponente E):**

Der Polymerisationsinhibitor wird für die Gewährleistung einer ausreichenden Lagerstabilität der Mischung der Komponenten B), D), E) und F) benötigt. Die Wirkung der Inhibitoren besteht meist darin, dass sie als Radikalfänger für die bei der Polymerisation auftretenden freien Radikale wirken. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxioxidantien" und die an dieser Stelle zitierten Literaturstellen verwiesen. Geeignete Inhibitoren umfassen u. a. ggf. substituierte Phenole, ggf. substituierte Hydrochinone, wie beispielsweise Hydrochinonmonomethylether (HQME), ggf. substituierte Chinone, ggf. substituierte Brenzcatechine, Tocopherol, tert.-Butylmethoxyphenol (BHA), Butylhydroxytoluol (BHT), Octylgallat, Dodecylgallat, Ascorbinsäure, ggf. substituierte aromatische Amine, ggf. substituierte Metallkomplexe eines aromatischen Amins, ggf. substituierte Triazine, organische Sulfide, organische Polysulfide, organische Dithiocarbamate, organische Phosphite und organische Phosphonate, Phenothiazin und 4-Hydroxy-2,2,6,6-tetramethylpiperidin-1-oxyl.
Ggf. substituierte Hydrochinone und ggf. substituierte Phenole werden bevorzugt eingesetzt. Besonders bevorzugt werden Hydrochinon, Hydrochinonmonomethylether und 4-Methyl-2,6-di-tert-butylphenol.
In der Regel sind 0,2 Gew.-% Inhibitor ausreichend, meist liegt der Anteil deutlich niedriger, beispielsweise bei 0,05 Gew.-% oder darunter. Die Topfzeit des Systems nach Zumischung der Komponenten A und C wird erfindungsgemäß über die Anquellung der Komponente A gesteuert. Höhere Anteile als 0,2 Gew.-% Inhibitor, z.B. 1 Gew.-% oder höher, die bei Systemen des Standes der Technik manchmal zur Verlängerung der Topfzeit eingesetzt werden, sind deshalb meist nicht notwendig, sollen aber nicht ausgeschlossen werden. Bevorzugt ist ein Gehalt von maximal 0,2 Gew.-%, insbesondere maximal 0,05 Gew.-%.

**Komponente F:**

Die Formulierung kann neben den beschriebenen Komponenten übliche partikuläre Füllstoffe, wie beispielsweise Titandioxyd, Ruß oder Siliziumdioxyd, Glas, Glasperlen, Glaspulver, Zement, Quarzsand, Quarzmehl, Sand, Korund, Steingut, Klinker, Schwerspat, Magnesia, Calciumkarbonat, Marmormehl oder Aluminiumhydroxyd, mineralische oder organische Pigmente und Hilfsstoffe enthalten.
Hilfsstoffe können beispielsweise sein: Weichmacher, Wasser, Verlaufshilfsmittel, Verdickungsmittel, Entschäumer, Haftmittel oder Netzmittel. Vorzugsweise ist neben eventuell für die Phlegmatisierung des Peroxids eingesetzten Weichmacher kein weiterer Weichmacher enthalten.
Die partikulären Füllstoffe weisen üblicherweise einen Korndurchmesser von ca. 0,001 mm bis ca. 6 mm auf.
Auf ein Gewichtsteil Polymer werden üblicherweise 0 bis 8 Gewichtsteile Füllstoffe eingesetzt.

**Das Mischungsverhältnis**

Das Mischungsverhältnis ist von der angestrebten Anwendung abhängig. Diese bestimmt die eingesetzte Menge der Komponenten A - F. Das Mischungsverhältnis der eingesetzten Komponenten ist bevorzugt so zu wählen, dass eine vollständige Polymerisation des gegebenen Systems erreicht wird. Insbesondere soll zweckmäßig eine ausreichende Menge eines Redoxinitiatorsystems zur Verfügung stehen, wobei der Aktivator zumindest überwiegend in

Form eines Emulsionspolymerisats (Komponente A) zur Verfügung gestellt wird.

Da der Anteil des polymerisierbaren Aktivators A e) in Komponente A in breiten Grenzen gewählt werden kann, besteht auch für die Einsatzmenge der Komponente A ein breiter Spielraum. So kann der Anteil der Komponente A zwischen 0,8 und 69,94 Gew.-% betragen, und selbst wiederum 0,1 bis 95 Gew.-% des polymerisierbaren Aktivators enthalten. In der Regel wird die Aktivatormenge auf den eingesetzten Anteil des Peroxids abgestimmt. Das Peroxid ist der Partner des Aktivators im Redoxsystem. Sein Anteil beträgt in der Regel zwischen 0,05 Gew.-% und 10 Gew.-%, bevorzugt 0,1 Gew.-% bis 5 Gew.-%. Meist wird ein Anteil von 0,5 Gew.-% -5 Gew.-% gewählt, bevorzugt 0,5 Gew.-% - 3, insbesondere 0,5 Gew.-% - 2 Gew.-%. Maßgebend für den Peroxidanteil und den Anteil der Komponente A ist, dass bei der vorgesehenen Anwendung eine im erwünschten Maße vollständige Polymerisation in der gewünschten Zeit erfolgt und das ausgehärtete System die auf den Einsatzzweck abgestimmte Performance leistet.

Der Anteil eines ethylenisch ungesättigten Monomeren (Komponente B) kann zwischen 30 Gew.-% und 99 Gew.-% betragen. Vorzugsweise beträgt er 40 Gew.-% - 90 Gew.-%, insbesondere 40 Gew.-% - 80 Gew.-%. Der Anteil eines Oligomeren bzw. Polymeren (Komponente D) beträgt 0 Gew.-% - 60 Gew.-%, vorzugsweise 0 Gew.-% - 40 Gew.-%, insbesondere 0 Gew.-% - 30 Gew.-%.

Ferner kann die Mischung zwischen 0 und 800 Gewichtsteile, bezogen auf die Summe von A - D zu 100 Gewichtsteilen, an Füllstoffen, Pigmenten und sonstigen Hilfsstoffen enthalten.

Bevorzugte Zwei- oder Mehrkomponenten-Systeme gemäß der Erfindung umfassen

A) 0,8 Gew.-% - 69,94 Gew.-% eines Polymerisats wie hierin oben beschrieben mit polymerfixierter Aktivatorkomponente;
B) 30 Gew.-% - 99,14 Gew.-% eines oder einer Mehrzahl ethylenisch ungesättigter Monomere;
C) 0,05 Gew.-% - 10 Gew.-% Peroxide; gegebenenfalls
D) 0 Gew.-% - 60 Gew.-% Oligomere;
E) 0,01 Gew.-% - 2 Gew.-% eines Polymerisationsinhibitors; und gegebenenfalls
F) 0 - 800 Gewichtsteile Hilfs- und Zusatzstoffe;

wobei die Summe der Bestandteile A) + B) + C) + D) + E)100 Gew.-% ergibt und sich die Menge von F) auf 100 Gewichtsteile der Summe A) + B) + C) + D) + E) bezieht.

Weiters sind auch Systeme bevorzugt enthaltend 5 bis 45 Gew.-% Komponente A),

40 Gew.-% bis 94,89 Gew.-% Komponente B),
0,1 Gew.-% bis 5 Gew.-% Komponente C),
0 Gew.-% - 30 Gew.-% Komponente D);
0,01 Gew.-% - 0,2 Gew.-% Komponente E)
und
0 bis 800 Gewichtsteile Komponente F),

wobei die Summe der Bestandteile A) + B) + C) + D) +E) 100 Gew.-% ergibt und sich die Menge von F) auf 100 Gewichtsteile der Summe A) + B) + C) + D) +E) bezieht.

Noch mehr bevorzugt sind Systeme enthaltend

5 Gew.-% bis 45 Gew.-% Komponente A),
50 Gew.-% bis 94,50 Gew.-% Komponente B),
0,5 Gew.-% bis 5 Gew.-% Komponente C),
0 Gew.-% Komponente D);
und
0 bis 800 Gewichtsteile Komponente F),

wobei die Summe der Bestandteile A) + B) + C) + D) + E)100 Gew.-% ergibt und sich die Menge von F) auf 100 Gewichtsteile der Summe A) + B) + C) + D) +E) bezieht Besonders bevorzugt beträgt der Gehalt der Komponente D) 0 Gew.-%.

Von Interesse für die Erfindung sind auch Systeme, bei denen die Komponente A in flüssiger Form vorliegt. Dies ermöglicht ein leichteres Mischen der einzelnen Komponenten vor der Anwendung. So kann Komponente A als wässrige Dispersion, wie sie durch Emulsionspolymerisation erhalten wird, ohne Isolierung des Polymeren eingesetzt werden oder nachträglich wieder in Wasser suspendiert werden. Solche Einsatzformen setzen voraus, dass Wasser in der eingebrachten Menge im System nicht stört.

Soll Wasser vermieden werden, kann es im Rahmen der Erfindung auch von Vorteil sein, lagerstabile flüssige oder pastöse Formulierungen von Komponente A durch den Einsatz eines nicht anquellenden Monomeren bzw. einer

Mischung nicht anquellender Monomere zu erreichen, die einen Teil der Komponente B darstellen. Lagerstabil bedeutet, dass ein eventueller Viskositätsanstieg so begrenzt ist, dass eine Mischung aller Komponenten vor der Verwendung möglich ist.

Von besonderem Interesse für die Erfindung sind auch Systeme, bei denen Peroxid C) und aminische Aktivatorkomponente (verkapselt im Polymerisat A) gemeinsam miteinander vorliegen. Dies ist überraschend, da solche Initiatorkomponenten in der Regel bis zur Anwendung getrennt voneinander zu lagern waren.

In besonders zweckmäßiger Ausgestaltung umfaßt die Erfindung ein System, welches dadurch gekennzeichnet ist, dass Komponente A) und Komponente C) gemeinsam gelagert werden und bis zur Anwendung des Systems wenigstens ein Bestandteil der Komponente B) getrennt von A) und C) gelagert wird, wobei das Quellvermögen des getrennt gelagerten Bestandteils der Komponente B) für das Polymerisat A) so hoch ist, dass der polymerfixierte Aktivator des Polymerisats A) mit der Komponente C) zur Umsetzung gelangen kann.

Dargestellt wird ein derartiges System durch Beimischung eines Peroxids, meist Benzoylperoxid, zu einer wäßrigen Polymerdispersion, in welcher eine polymerisierbare Aktivatorkomponente im Polymeren verkapselt ist, vorzugsweise durch einen Kern-/Schale-Aufbau. Das System bestehend aus einer wäßrigen Dispersion mit verkapselter, polymergebundener Aktivatorkomponente und einem in der wäßrigen Phase befindlichen peroxidischen Initiator ist damit lagerstabil, da ein Kontakt zwischen Peroxid und Amin unterbunden ist. Zur Nutzung eines derartigen lagerstabilen Initiatorsystems zur Polymerisation wird eine Anquellung der Polymerteilchen mit geeigneten Monomeren herbeigeführt.

Im Rahmen der Erfindung kann es auch von Vorteil sein, eine Lagerstabilität des Zwei- oder Mehrkomponentensystems nicht durch die wäßrige Phase zu erreichen, sondern durch den Einsatz eines nicht anquellenden Monomeren bzw. einer Mischung nicht anquellender Monomere. Die nicht anquellenden Monomeren stellen einen Teil der Komponente B dar.

Ein besonderes erfindungsgemäßes System ist dadurch gekennzeichnet, dass Komponente A), ein Teil von Komponente B) und Komponente C) gemeinsam gelagert werden, wobei der Teil der Komponente B) so gewählt wird, dass das Quellvermögen dieser Bestandteile der Komponente B) für das Polymerisat A) so gering ist, dass der polymerfixierte Aktivator des Polymerisats A) mit der Komponente C) nicht zur Umsetzung gelangen kann. Wesentlich ist aber, dass das Quellvermögen der Gesamtheit der Monomeren der Komponente B nach Mischung aller Komponenten ausreichend hoch ist, um die Polymerisation des Systems auszulösen.

Dargestellt wird ein solches System beispielsweise durch Isolierung der weiter oben beschriebenen Emulsionspolymerisate bevorzugt durch Sprühtrocknung. Das so als Feststoff erhaltene Polymerisat A), in welchem die polymerfixierte Aktivatorkomponente verkapselt ist, wird anschließend in einem das Polymer nicht anquellenden oder nicht anlösenden Monomer dispergiert. Es erfolgt die Beimischung eines oder mehrerer Peroxide C), bevorzugt z.B. Benzoylperoxid, zu dieser Abmischung, in welcher eine polymerisierbare Aktivatorkomponente im Polymeren verkapselt ist. Durch die Polymeranbindung wird eine mögliche Oberflächenbeladung des Polymerteilchens durch den Aktivator praktisch ausgeschlossen. Das System bestehend bevorzugt aus einem Kern/Schale-Polymerisat mit verkapselter. Polymergebundener Aktivatorkomponente und einem in der nicht anquellenden Monomerphase befindlichen Initiator ist damit lagerstabil, da ein Kontakt zwischen Komponente C) und Aktivator im Polymerisat A) unterbunden ist.

Zur Nutzung eines derartigen lagerstabilen Initiatorsystems zur Polymerisation wird eine Anquellung der Polymerteilchen mit geeigneten Monomeren herbeigeführt, welche dem System dann zugesetzt werden. Die Aktivatorkomponente wird freigesetzt, und eine Aushärtung dieser Mischung einschließlich der nicht anquellenden Monomere wird möglich. Der Anquellwiderstand lässt sich insbesondere wie hierin oben beschrieben einstellen.

**Anwendungen:**

Das System eignet sich grundsätzlich für alle Zwei-Komponentensysteme wie Klebstoffe, Gießharze, Bodenbeschichtungen und sonstige Reaktivbeschichtungen, Abdichtmassen, Imprägniermassen, Einbettmassen, Reaktivdübel, Dentalmassen, Herstellung von künstlichem Marmor oder anderen Kunststeinen, poröse Kunststoffformen für keramische Objekte und ähnliche Anwendungen. Es ist auch geeignet für den Einsatz in ungesättigten Polyesterharzen und deren typischen Anwendungen.

Besonders bevorzugt ist die Anwendung des beschriebenen Zwei- oder Mehrkomponentensystems in Klebstoffen, Gießharzen, Bodenbeschichtungen, Massen für Reaktivdübel, Dentalmassen oder Abdichtmassen.

In einer Anwendung als Gießharz kann ein hoher Polymeranteil (Komponente A), beispielsweise zwischen 30 Gew.-% und 70 Gew.-%, vorteilhaft sein. Der Anteil des Aktivators in Komponente A kann dann beispielsweise auf 0,1 Gew.-% bis 5 Gew.-% bezogen auf Komponente A beschränkt werden. Die Komponenten B und D zusammengenommen liegen dann zwischen 69,9 Gew.-% und 30 Gew.-%. Der Peroxidanteil beträgt vorzugsweise 0,1 Gew.-% bis 5 Gew.-%.

Im Bereich hochvernetzter Systeme kann es sinnvoll sein, den Gehalt an Polymer (Komponente A) zu begrenzen und nur als Träger eines Aktivators einzusetzen. Der Anteil der Komponente A ist daher vorzugsweise entsprechend gering und liegt beispielsweise zwischen 1 Gew.-% und 10 Gew.-%. Der Anteil des in Komponente A polymerfixierten Aktivators ist entsprechend hoch zu wählen und kann 10 Gew.-% oder sogar bis zu 60 Gew.-%, in Einzelfällen auch bis

zu 95 Gew.-% bezogen auf Komponente A betragen. Die Komponenten B und D zusammengenommen liegen dann zwischen 98,9 und 90 Gew.-%. Der Peroxidanteil beträgt vorzugsweise 0,1 Gew.-% bis 5 Gew.-%.

Die nachfolgenden Beispiele und Vergleichsbeispiele dienen zur weiteren Erläuterung der Erfindung.

Herstellung der Emulsionspolymerisate

Alle Emulsionspolymerisate wurden im Zulaufverfahren hergestellt.

Die Vorlage wurde im Reaktionsgefäß 5 min bei 80 °C gerührt. Anschließend wurde der restliche Zulauf 1 über einen Zeitraum von 3 h und Zulauf 2 über einen Zeitraum von 1 h zugegeben. Die Zuläufe 1 und 2 wurden vor Zugabe zur Reaktionsmischung emulgiert. Es wurde demineralisiertes Wasser verwendet.
Die Ansätze sind in Tabelle 1 aufgeführt.

Tabelle 1

| Versuch Nr. | Vorlage | Zulauf 1 | Zulauf 2 | Charakterisierung |
|---|---|---|---|---|
| 1 | 341,0 g Wasser 0,72 g 10%ige C15-Paraffinsulfonat, Na-Salz-Lösung<br>6,0 g 10%ige 4,4'-Azobis-(4-cyanovaleriansäure),Na-Salz-Lösung | 12,0 g 10%ige C15-Paraffinsulfonat, Na-Salz-Lösung<br>24,0 g 10%ige 4,4'-Azobis-(4-cyanovaleriansäure),Na-Salz-Lösung<br>400,0 g MMA<br>400,0 g Wasser | 12,0 g 10%ige C15-Paraffinsulfonat, Na-Salz-Lösung<br>24,0 g 10%ige 4,4'-Azobis-(4-cyanovaleriansäure),Na-Salz-Lösung<br>380,0 g MMA<br>20,0 g MAS<br>400,0 g Wasser | FG:38,8%<br>mittlere Teilchengröße, Gerät Mastersizer: 158 nm<br>pH-Wert: 6,1 |
| 2 | 341,0 g Wasser 0,72 g 10%ige C15-Paraffinsulfonat, Na-Salz-Lösung<br>6,0 g 10%ig 4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>396,0 g MMA<br>4,13g2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:39,0%<br>mittlere Teilchengröße, Gerät Mastersizer: 171 nm<br>pH-Wert: 6,1 |
| 3 | 341,5 g Wasser 0,72 g 10%ige C15-Paraffinsulfonat, Na-Salz-Lösung<br>6,0 g 10%ig4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>392,0 g MMA<br>8,20 g 2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:38,7%<br>mittlere Teilchengröße, Gerät Mastersizer: 176 nm<br>pH-Wert: 6,0 |
| 4 | 341,0 g Wasser<br>0,72 g 10%ige C15-Paraffinsulfonat, Na-Salz-Lösung<br>6,0 g 10%ige 4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>388,0 g MMA<br>12,38 g 2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:38,9%<br>mittlere Teilchengröße, Gerät Mastersizer: 189 nm<br>pH-Wert: 6,1 |

(fortgesetzt)

| Versuch Nr. | Vorlage | Zulauf 1 | Zulauf 2 | Charakterisierung |
|---|---|---|---|---|
| | 341,0 g Wasser<br>0,72 g 10%ige C15-Paraffinsulfonat, Na-Salz-Lösung<br>6,0 g 10%ig4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>384,0 g MMA<br>16,50 g 2-N-(Ethylanilino)-ethylmethacrylat<br>400,0 g Wasser | 12,0g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:38,6%<br>mittlere Teilchengröße, Gerät Mastersizer: 167 nm<br>pH-Wert: 5,9 |
| 6 | 342,2 g Wasser<br>0,72 g 10%ige C15-Paraffinsulfonat, Na-Salz-Lösung<br>6,0 g 10%ige 4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>376,0 g MMA<br>24,80 g 2-N-(Ethylanilino)-ethylmethacrylat<br>400,0 g Wasser | 12,0g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:39,1%<br>mittlere Teilchengröße, Gerät Mastersizer: 183 nm<br>pH-Wert: 6,1 |
| 7 | 342,2 g Wasser<br>0,72g 10%ige C15-Paraffinsulfonat, Na-Salz-Lösung<br>6,0 g 10%ige 4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäur e), Na-Salz-Lösung<br>368,0 g MMA<br>33,03 g 2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:39,0%<br>mittlere Teilchengröße, Gerät Mastersizer: 165 nm<br>pH-Wert: 6,3 |
| 8 | 342,2 g Wasser<br>0,72 g 10%ige C15-Paraffinsulfonat, Na-Salz-Lösung<br>6,0 g 10%ige 4,4'-Azobis-(4-cyanovaleriansäure), Na-Salz-Lösung | 12,0 g 10%ige C15-Paraffinsulfonat, Na-Salz-Lösung<br>24,0 g 10%ige 4,4'-Azobis-(4-cyanovaleriansäure), Na-Salz-Lösung<br>360,0 g MMA<br>41,30 g 2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:38,8%<br>mittlere Teilchengröße, Gerät Mastersizer: 236 nm<br>pH-Wert: 6,0 |

(fortgesetzt)

| Versuch Nr. | Vorlage | Zulauf 1 | Zulauf 2 | Charakterisierung |
|---|---|---|---|---|
| 9 | 343,9 g Wasser<br>0,72 g 10%ige C15-Paraffinsulfonat, Na-Salz-Lösung<br>6,0 g 10%ige 4,4'-Azobis-(4-cyanovaleriansäure), Na-Salz-Lösung | 12,0g 10%ige C15-Paraffinsulfonat, Na-Salz-Lösung<br>24,0 g 10%ige 4,4'-Azobis-(4-cyanovaleriansäure), Na-Salz-Lösung<br>340,0 g MMA<br>62,40 g 2-N-(Ethylanilino)-ethylmethacrylat<br>400,0 g Wasser | 12,0 g 10%ige C15-Paraffinsulfonat, Na-Salz-Lösung<br>24,0 g 10%ige 4,4'-Azobis-(4-cyanovaleriansäure), Na-Salz-Lösung<br>380,0 g MMA<br>20,0 g MAS<br>400,0 g Wasser | FG:38,7%<br>mittlere Teilchengröße, Gerät Mastersizer: 198 nm<br>pH-Wert: 6,1 |
| 10 | 262,5 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>240,0 g MMA<br>62,10g 2-N-(Ethylanilino)-ethylmethacrylat<br>300,0 g Wasser | 9,0 g 10%ige C15-Paraffinsulfonat, Na-Salz-Lösung<br>18,0g 10%ige 4,4'-Azobis-(4-cyanovaleriansäure), Na-Salz-Lösung<br>285,0 g MMA<br>15,0 g MAS<br>300,0 g Wasser | FG:38,7%<br>mittlere Teilchengröße, Gerät Mastersizer: 289 nm<br>pH-Wert: 5,3 |
| 11 | 263,4 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>225,0 g MMA<br>77,60 g 2-N-(Ethylanilino)-ethylmethacrylat<br>300,0g Wasser | 9,0g 10%ige C15-Paraffinsulfonat, Na-Salz-Lösung<br>18,0 g 10%ige 4,4'-Azobis-(4-cyanovaleriansäure), Na-Salz-Lösung<br>285,0 g MMA<br>15,0 g MAS<br>300,0 g Wasser | FG:38,0%<br>mittlere Teilchengröße, Gerät Mastersizer: 283 nm<br>pH-Wert: 5,2 |
| 12 | 264,1 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>210,0g MMA<br>93,1 g 2-N-(Ethylanilino)-ethylmethacrylat<br>300,0 g Wasser | 9,0 g 10%ige C15-Paraffinsulfonat, Na-Salz-Lösung<br>18,0 g 10%ige 4,4'-Azobis-(4-cyanovaleriansäure), Na-Salz-Lösung<br>285,0 g MMA<br>15,0 g MAS<br>300,0 g Wasser | FG:38,9%<br>mittlere Teilchengröße, Gerät Mastersizer: 340 nm<br>pH-Wert: 6,8 |

(fortgesetzt)

| Versuch Nr. | Vorlage | Zulauf 1 | Zulauf 2 | Charakterisierung |
|---|---|---|---|---|
| 13 | 264,9 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>195,0 g MMA<br>108,0 g 2-N-(Ethylanilino)-ethylmethacrylat<br>300,0 g Wasser | 9,0 g 10%ige C15-Paraffinsulfonat, Na-Salz-Lösung<br>18,0 g 10%ige 4,4'-Azobis-(4-cyanovaleriansäure), Na-Salz-Lösung<br>285,0 g MMA<br>15,0 g MAS<br>300,0 g Wasser | FG:39,3%<br>mittlere Teilchengröße, Gerät Mastersizer: 161 nm<br>pH-Wert: 5,2 |
| 14 | 177,05 g Wasser<br>0,36 g 10%ige C15-Paraffinsulfonat, Na-Salz-Lösung<br>3,0 g 10%ige 4,4'-Azobis-(4-cyanovaleriansäure), Na-Salz-Lösung | 6,0 g 10%ige C15-Paraffinsulfonat, Na-Salz-Lösung<br>12,0 g 10%ige 4,4'-Azobis-(4-cyanovaleriansäure), Na-Salz-Lösung<br>120,0 g MMA<br>82,70 g 2-N-(Ethylanilino)-ethylmethacrylat<br>200,0 g Wasser | 6,0 g 10%ige C15-Paraffinsulfonat, Na-Salz-Lösung<br>12,0 g 10%ige 4,4'-Azobis-(4-cyanovaleriansäure), Na-Salz-Lösung<br>190,0 g MMA<br>10,0 g MAS<br>200,0 g Wasser | FG:38,7%<br>mittlere Teilchengröße, Gerät Mastersizer: 173 nm<br>pH-Wert: 5,3 |
| 15 | 177,6 g Wasser<br>0,36 g 10%ige C15-Paraffinsulfonat, Na-Salz-Lösung<br>3,0 g 10%ige 4,4'-Azobis-(4-cyanovaleriansäure), Na-Salz-Lösung | 6,0 g 10%ige C15-Paraffinsulfonat, Na-Salz-Lösung<br>12,0 g 10%ige 4,4'-Azobis-(4-cyanovaleriansäure), Na-Salz-Lösung<br>110,0g MMA<br>93,10g 2-N-(Ethylanilino)-ethylmethacrylat<br>200,0g Wasser | 6,0 g 10%ige C15-Paraffinsulfonat, Na-Salz-Lösung<br>12,0 g 10%ige 4,4'-Azobis-(4-cyanovaleriansäure), Na-Salz-Lösung<br>190,0 g MMA<br>10,0 g MAS<br>200,0 g Wasser | FG:38,7%<br>mittlere Teilchengröße, Gerät Mastersizer: 164 nm<br>pH-Wert: 5,4 |
| 16 | 260,1 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>210,0 g MMA<br>92,9 g 2-N-(Ethylanilino)-ethylmethacrylat<br>300,0 g Wasser | 9,0 g 10%ige C15-Paraffinsulfonat, Na-Salz-Lösung<br>18,0 g 10%ige 4,4'-Azobis-(4-cyanovaleriansäure), Na-Salz-Lösung<br>285,0 g MMA<br>15,0 g MAA<br>300,0 g Wasser | FG:38,2%<br>mittlere Teilchengröße, Gerät Mastersizer: 229 nm<br>pH-Wert: 6,1 |

(fortgesetzt)

| Versuch Nr. | Vorlage | Zulauf 1 | Zulauf 2 | Charakterisierung |
|---|---|---|---|---|
| 17 | 260,1 g Wasser<br>0,54g 10%ige C15-Paraffinsulfonat, Na-Salz<br>4,5 g 10%ige 4,4'-Azobis-(4-cyanovaleriansäure), Na-Salz-Lösung | 9,0g 10%ige C15-Paraffinsulfonat, Na-Salz<br>18,0 g 10%ige 4,4'-Azobis-(4-cyanovaleriansäure), Na-Salz-Lösung<br>210,0 g MMA<br>92,9 g 2-N-(Ethylanilino)-ethylmethacrylat<br>300,0 g Wasser | 9,0 g 10%ige C15-Paraffinsulfonat, Na-Salz<br>18,0 g 10%ige 4,4'-Azobis-(4-cyanovaleriansäure), Na-Salz-Lösung<br>270,0 g MMA<br>15,0 g MAA<br>15,0 g MAS<br>300,0 g Wasser | FG:39,0%<br>mittlere Teilchengröße, Gerät Mastersizer: 255 nm<br>pH-Wert: 5,5 |
| 18 | 260,1 g Wasser<br>0,54 g 10%ige C15-Paraffinsulfonat, Na-Salz-Lösung<br>4,5 g 10%ige 4,4'-Azobis-(4-cyanovaleriansäure), Na-Salz-Lösung | 9,0 g 10%ige C15-Paraffinsulfonat, Na-Salz-Lösung<br>18,0 g 10%ige 4,4'-Azobis-(4-cyanovaleriansäure), Na-Salz-Lösung<br>210,0 g MMA<br>92,9 g 2-N-(Ethylanilino)-ethylmethacrylat<br>300,0 g Wasser | 9,0 g 10%ige C15-Paraffinsulfonat, Na-Salz-Lösung<br>18,0 g 10%ige 4,4'-Azobis-(4-cyanovaleriansäure), Na-Salz-Lösung<br>285,0 g MMA<br>15,0 g MAS<br>300,0 g Wasser | FG:39,1 %<br>mittlere Teilchengröße, Gerät Mastersizer: 227 nm<br>pH-Wert: 5,3 |

In Tabelle 1 verwendete Abkürzungen:

MMA: Methylmethacrylat
MAS: Methacrylsäure
FG: Feststoffgehalt

Herstellung einer Monomer-Polymer-Mischung und Bestimmung der Anquellzeit

20 g (= 40 Gew.-%) des jeweiligen Polymeren (Komponente A) werden in einem Becher (0,2 l) vorgelegt. 30 g (= 60 Gew.-%) eines ethylenisch ungesättigten Monomeren bzw. einer Monomermischung (Komponente B) zugegeben und mit einem Holzspatel solange gerührt bis die Mischung als nicht mehr verarbeitbar angesehen wird. Diese Zeit wird als Anquell- bzw. Topfzeit angegeben.

Die Ergebnisse sind in Tabelle 2 aufgeführt. Die Versuche ohne Aushärtung zeigen, wie durch Einbau von polaren Monomeren der Anquellwiderstand erhöht werden kann.

Gelierzeitmessung mit dem GELNORM - Gel Timer

Gerätebeschreibung:

Der GELNORM-Gel-Timer ist ein automatisches Gerät zur Bestimmung der Gelierzeit von Reaktionsharzen in Anlehnung an die DIN 16945, Blatt 1 und DIN 16916.

Geräteaufbau:

Klemmhalter, Rändelschraube, Meßstempel, Mikroschalter, Haltefeder, Reagenzglas, Reagenzglashalterung

Durchführung:

Es wurde eine Mischung aus 5 g Pulver und 7,5 g Monomer hergestellt. Die Mischung wurde ca. 1 Min. mit einem Holzspatel gerührt und in ein Reagenzglas 160mm x Ø 16mm (Eigengewicht ca. 10g) eingefüllt. Das Gesamtgewicht von Reagenzglas und Prüfmischung sollte stets 22 g betragen, um eine gute Reproduzierbarkeit der Messergebnisse zu gewährleisten.
Das Reagenzglas inklusive Haltefeder und Prüfmischung wurde in die Halterung des Meßkopfes gestellt und gleichzeitig die Haltefeder am Mikroschalter eingehängt. Anschließend wurde der Meßstempel in die Mischung eingetaucht und am Klemmhalter befestigt. Danach wurde der Versuch bei Raumtemperatur gestartet.
Beim Erreichen des Gelierpunktes wurde die Zeitmessung mittels Mikroschalter durch das Hochziehen des Reagenzglases gestoppt. Das Gerät hat eine Ablesegenauigkeit von einer Sekunde.

Tabelle 2

| Versuch Nr. | Zusammensetzung | | Monomer Komponente | Anquellzeit [min] | Gelierzeit [min] | Polyzeit [min] | Spitzen-temp.[°C ] |
|---|---|---|---|---|---|---|---|
| | Kern 50% | Schale 50% | | | | | |
| 1 | 100% MMA | 95% MMA<br>5% MAS | THFMA | 31 | 17 | - | - |
| 2 | 99% MMA<br>1% 2-(N-Ethylanilino)-ethylmethacrylat | 95% MMA<br>5% MAS | THFMA | 20 | 13 | 144 | 26,5 |
| 3 | 98% MMA<br>2% 2-(N-Ethylaniliono)-ethylmethacrylat2-(N-Ethylaniliono)-ethylmethacrylat | 95% MMA<br>5% MAS | THFMA | 24 | 37 | 1440 | 24 |
| 4 | 97% MMA<br>3% 2-(N-Ethylaniliono)-ethylmethacrylat | 95% MMA<br>5% MAS | THFMA | 30 | 47 | 215 | 47 |
| 5 | 96% MMA<br>4% 2-(N-Ethylaniliono)-ethylmethacrylat | 95% MMA<br>5% MAS | THFMA | 50 | 38 | 130 | 61 |
| 6 | 94% MMA<br>6% 2-(N-Ethylaniliono)-ethylmethacrylat | 95% MMA<br>5% MAS | THFMA | 34 | 43 | 101 | 68 |
| 7 | 92% MMA<br>8% 2-(N-Ethylaniliono)-ethylmethacrylat | 95% MMA<br>5% MAS | THFMA | 30 | 38 | 79 | 70 |
| 8 | 90% MMA<br>10% 2-(N-Ethylaniliono)-ethylmethacrylat | 95% MMA<br>5% MAS | THFMA | 60 | 19 | 123 | 80 |
| 9 | 85% MMA<br>15% 2-(N-Ethylaniliono)-ethylmethacrylat | 95% MMA<br>5% MAS | THFMA | 60 | 17 | 98 | 97 |
| 10 | 80% MMA | 95% MMA | THFMA | 60 | 39 | 60 | 99 |

(fortgesetzt)

| Versuch Nr. | Zusammensetzung | | Monomer Komponente | Anquellzeit [min] | Gelierzeit [min] | Polyzeit [min] | Spitzen-temp.[°C ] |
|---|---|---|---|---|---|---|---|
| | 20% 2-(N-Ethylaniliono)-ethylmethacrylat | 5% MAS | | | | | |
| 11 | 75% MMA<br>25% 2-(N-Ethylaniliono)-ethylmethacrylat | 95% MMA<br>5% MAS | THFMA | 36 | 52 | 66 | 102 |
| 12 | 70% MMA<br>30% 2-(N-Ethylaniliono)-ethylmethacrylat | 95% MMA<br>5% MAS | THFMA | 43 | 63 | 73 | 112 |
| 13 | 65% MMA<br>35% 2-(N-Ethylaniliono)-ethylmethacrylat | 95% MMA<br>5 % MAS | THFMA | 15 | 21 | 35 | 116 |
| 14 | 60% MMA<br>40% 2-(N-Ethylaniliono)-ethylmethacrylat | 95% MMA<br>5% MAS | THFMA | 12 | 22 | 26 | 114 |
| 15 | 55% MMA<br>45% 2-(N-Ethylaniliono)-ethylmethacrylat | 95% MMA<br>5% MAS | THFMA | 21 | 20 | 46 | 111 |
| 16 | 70% MMA<br>30% 2-(N-Ethylaniliono)-ethylmethacrylat | 95% MMA<br>5% MAA | THFMA | 125 | nicht messbar | 188 | 80 |
| 17 | 70% MMA<br>30% 2-(N-Ethylaniliono)-ethylmethacrylat | 95% MMA<br>5% MAA<br>5% MAS | THFMA | >450 | nicht messbar | >450 | 22 |
| 18 | 70% MMA<br>30% 2-(N-Ethylaniliono)-ethylmethacrylat | 95% MMA<br>5% MAS | THFMA | 61 | **61'** | 90 | 100 |
| 19 | 70% MMA<br>30% 2-(N-Ethylaniliono)-ethylmethacrylat | 98% MMA<br>2% MAS | 1,4-BDDMA:HPMA=1:1 | 20 | **36** | 24 | 144 |

In Tabelle 2 verwendete Abkürzungen:

MMA: Methylmethacrylat
MAS: Methacrylsäure
MAA: Methacrylamid
THFMA: Tetrahydrofurfurylmethacrylat
1,4-BDDMA: 1,4 Butandioldimethacrylat
HPMA: Hydroxypropylmethacrylat

Aushärtung von Filmen in dünner Schicht:

Durchführung: 5 g des jeweiligen Polymeren (Komponente A) werden in einem Becher (0,2 l) vorgelegt und mit unterschiedlichen Mengen MMA versetzt. Die Mischungen wurden mit jeweils 1,3 g BP-50-FT versetzt.
Folgende Mischungsverhältnisse wurden untersucht:

| Polymer (Komponente A) | Methylmethacrylat | Mischungsverhältnis (Gew.-%/Gew.-%) | BP-50-FT |
|---|---|---|---|
| 5 g | 11,65 g | 30:70 | 1,3 g |
| 5 g | 15,00 g | 25:75 | 1,3 g |
| 5 g | 20,00 | 20:80 | 1,3 g |

Die erzeugten Mischungen wurden zu Filmen gerakelt. Die Schichtdicke variierte dabei zwischen 0,85 mm und 0,07 mm. Die Aushärtung der Filme erfolgte an Luft und war innerhalb von 60 min vollständig abgeschlossen.

Bestimmung der Polymerisationszeiten:

Polymersiationsverfahren: Benzoylperoxid BP-50-FT (BP-50-FT ist ein weißes fliessfähiges Pulver, Gehalt 50 Masse-% Dibenzoylperoxid, mit einem Phthalsäureester phlegmatisiert) wird in zum Aktivator äquimolaren Mengen mit den Monomeren B und Komponente A gemischt.
Alle Polymerisationen wurden im gleichen Mischungsverhältnis, wie schon bei der Bestimmung der Topfzeit beschrieben durchgeführt.
Die Polymerisationszeit ist definiert als die Zeit, die ein Ansatz vom Polymerisationsstart (Zugabe der Initiatoren), bis zum Erreichen der Polymerisationsspitzentemperatur benötigt. Als Ergebnis werden die benötigte Zeit und die Spitzentemperatur angegeben. Die Messung erfolgt mittels Kontaktthermometer unter Aufzeichnung des Temperaturverlaufs.

## Patentansprüche

**1.** Emulsionspolymerisat erhältlich durch Polymerisation einer Mischung aufweisend

a) 5 Gew.-% bis 99,9 Gew.-% eines oder einer Mehrzahl von Monomeren mit einer Wasserlöslichkeit < 2 Gew.-% bei 20 °C ausgewählt aus der Gruppe bestehend aus monofunktionellen (Meth)acrylatmonomeren, Styrol und Vinylestern;
b) 0 Gew.-% bis 70 Gew.-% eines oder einer Mehrzahl mit den Monomeren a) copolymerisierbaren Monomeren;
c) 0 Gew.-% bis 20 Gew.-% einer oder einer Mehrzahl zweifach oder mehrfach vinylisch ungesättigter Verbindungen;
d) 0 Gew.-% bis 20 Gew.-% eines oder einer Mehrzahl polarer Monomere mit einer Wasserlöslichkeit > 2 Gew.-% bei 20°C; und
e) 0,1 - 95 Gew.-% wenigstens eines Aktivators,

wobei die Komponenten a) bis e) zusammen 100 Gew.-% der polymerisierbaren Bestandteile der Mischung ergeben, **dadurch gekennzeichnet, dass**

e1) der Aktivator eine Verbindung der Formel I ist,

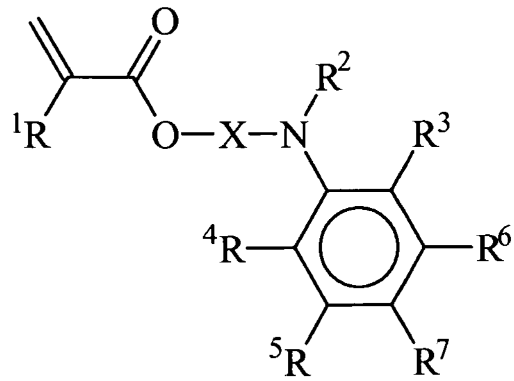

(I)

worin

- $R^1$ Wasserstoff oder Methyl ist;
- X eine lineare oder verzweigte Alkandiylgruppe mit 1 bis 18 Kohlenstoffatomen ist, die ein oder mehrfach mit Hydroxylgruppen und / oder mit $C_1$ - $C_4$ Alkoxygruppen substituiert sein kann;
- $R^2$ Wasserstoff oder einen linearen oder verzweigten Alkylrest mit 1 bis 12 Kohlenstoffatomen bedeutet, der gegebenenfalls ein oder mehrfach mit Hydroxylgruppen oder $C_1$ - $C_4$ -Alkoxygruppen substituiert ist, wobei die Hydroxylgruppen partiell mit (Meth)acrylsäure verestert sein können;
- $R^3$, $R^4$, $R^5$, $R^6$ und $R^7$ unabhängig voneinander Wasserstoff oder eine lineare oder verzweigte Alkyl- oder Alkoxygruppe mit 1 bis 8 Kohlenstoffatomen bedeuten, die ein oder mehrfach mit Hydroxylgruppen substituiert sein können; und wobei gegebenenfalls zwei der Reste $R^3$ bis $R^7$ miteinander zu einem fünf- bis siebengliedrigen Ring verbunden sind und gegebenenfalls ein kondensiertes aromatisches Ringsystem mit dem Phenylrest bilden;

und dass
e2) der Aktivator e) über kovalente Bindungen in das Emulsionspolymerisat eingebaut ist

und dass
die Bestandteile a) bis e) als Kern-Schale-Polymerisate eingesetzt werden.

2. Polymerisat nach Anspruch 1, **dadurch gekennzeichnet, dass** $R^1$ Methyl ist.

3. Polymerisat nach Anspruch 1 oder Anspruch 2, **dadurch gekennzeichnet, dass** X eine Ethylengruppe $-CH_2-CH_2-$ oder eine 2-Hydroxypropylengruppe $-CH_2-CH(OH)-CH_2-$ ist.

4. Polymerisat nach einem der vorhergehenden Ansprüche, **dadurch gekennzeichnet, dass** $R^2$ ausgewählt ist aus der Gruppe bestehend aus Methyl, Ethyl und 2-Hydroxyethyl.

5. Polymerisat nach einem der vorhergehenden Ansprüche, **dadurch gekennzeichnet, dass** einer der Reste $R^3$ bis $R^7$ Methyl ist während die verbleibenden vier Reste Wasserstoff sind oder zwei der Reste $R^3$ bis $R^7$ Methyl sind während die verbleibenden drei Reste Wasserstoff sind.

6. Polymerisat nach einem der vorhergehenden Ansprüche, **dadurch gekennzeichnet, dass** a) aus ein oder mehreren Methacrylatmonomeren und/oder Acrylatmonomeren besteht.

7. Polymerisat nach einem der vorhergehenden Ansprüche, **dadurch gekennzeichnet, dass** e) in einer Menge von 10-60 Gew.-%, vorzugsweise 20-50 Gew. %, vorliegt.

8. Polymerisat nach Anspruch 6, **dadurch gekennzeichnet, dass** a) Methylmethacrylat ist.

9. Verfahren zur Herstellung eines Emulsionspolymerisats, bei welchem man die Bestandteile a) bis e) gemäß den Ansprüchen 1 bis 8 in wäßriger Emulsion polymerisiert.

**10.** Verfahren nach Anspruch 9, bei welchem man nach Art einer Kern-Schale-Polymerisation die Bestandteile a) bis e) in einer ersten Stufe als Kern polymerisiert und daran anschließend in wenigstens einer weiteren Stufe als Schale eine Mischung der Bestandteile a) bis d).

**11.** Verfahren nach Anspruch 9, **dadurch gekennzeichnet, dass** man die Bestandteile a) bis e) für den Kern und die Bestandteile a) bis d) für die Schale so wählt, dass im resultierenden Polymerisat die Glastemperatur $T_{GS}$ mindestens einer Schale größer als die Glastemperatur $T_{GK}$ des Kerns ist, wobei die Glastemperaturen $T_G$ nach EN ISO 11357 bestimmt werden.

**12.** Verfahren nach Anspruch 11, **dadurch gekennzeichnet, dass** man die Bestandteile a) bis d) für die Schale so wählt, dass im resultierenden Polymerisat die Glastemperatur $T_{GS}$ mindestens einer Schale größer als 100 °C ist, wobei die Glastemperatur $T_{GS}$ nach EN ISO 11357 bestimmt wird.

**13.** Durch ein Redoxinitiatorsystem härtendes Zwei- oder Mehrkomponenten-System mit steuerbarer Topfzeit aufweisend ein Polymerisat gemäß den Ansprüchen 1 bis 8 oder ein nach einem Verfahren gemäß den Ansprüchen 9 bis 12 erhaltenes Polymerisat.

**14.** Zwei- oder Mehrkomponenten-System nach Anspruch 13 umfassend

A) 0,8 - 69,94 Gew.-% eines Polymerisats gemäß den Ansprüchen 1 bis 8 oder erhältlich nach dem Verfahren gemäß Ansprüchen 9 bis 12;
B) 30 - 99,14 Gew.-% eines oder einer Mehrzahl ethylenisch ungesättigter Monomere;
C) 0,05 - 10 Gew.-% Peroxide; gegebenenfalls
D) 0 - 60 Gew.-% ungesättigte Oligomere;
E) 0,01 - 2 Gew.-% eines Polymerisationsinhibitors; und gegebenenfalls
F) 0 - 800 Gewichtsteile Hilfs- und Zusatzstoffe;

wobei die Summe der Bestandteile A) + B) + C) + D) + E) 100 Gew.-% ergibt und sich die Menge von F) auf 100 Gewichtsteile der Summe A) + B) + C) + D) + E) bezieht.

**15.** System nach Anspruch 14, enthaltend

5 bis 45 Gew.-% Komponente A),
40 bis 94,89 Gew.-% Komponente B),
0,1 bis 5 Gew.-% Komponente C),
0-30 Gew.-% Komponente D);
0,01 - 0,2 Gew.-% Komponente E)
und
0 bis 800 Gewichtsteile Komponente F),

wobei die Summe der Bestandteile A) + B) + C) + D) +E) 100 Gew.-% ergibt und sich die Menge von F) auf 100 Gewichtsteile der Summe A) + B) + C) + D) + E) bezieht

**16.** System nach Anspruch 14, enthaltend

5 bis 45 Gew.-% Komponente A),
50 bis 94,50 Gew.-% Komponente B),
0,5 bis 5 Gew.-% Komponente C),
0 Gew.-% Komponente D);
und
0 bis 800 Gewichtsteile Komponente F),

wobei die Summe der Bestandteile A) + B) + C) + D) + E)100 Gew.-% ergibt und sich die Menge von F) auf 100 Gewichtsteile der Summe A) + B) + C) + D) +E) bezieht.

**17.** System nach einem der Ansprüche 13 bis 16,
**dadurch gekennzeichnet, dass**
Komponente A) und Komponente C) gemeinsam gelagert werden und bis zur Anwendung des Systems wenigstens

ein Bestandteil der Komponente B) getrennt von A) und C) gelagert wird, wobei das Quellvermögen des getrennt gelagerten Bestandteils der Komponente B) für das Polymerisat A) so hoch ist, dass der polymerfixierte Aktivator des Polymerisats A) mit der Komponente C) zur Umsetzung gelangen kann.

**18.** System nach einem der Ansprüche 13 bis 16,
**dadurch gekennzeichnet, dass**
Komponente A), ein Teil der Komponente B) und Komponente C) gemeinsam gelagert werden, wobei der Teil der Komponente B) so gewählt wird, dass das Quellvermögen dieses Bestandteils der Komponente B) für das Polymerisat A) so gering ist, dass der polymerfixierte Aktivator des Polymerisats A) mit der Komponente C) nicht zur Umsetzung gelangen kann.

**19.** System nach einem der Ansprüche 13 bis 16,
**dadurch gekennzeichnet, dass**
Komponente A) und ein Teil der Komponente B) gemeinsam gelagert werden, wobei der Teil der Komponente B) so gewählt wird, dass das Quellvermögen dieses Bestandteils der Komponente B) für das Polymerisat A) so gering ist, dass kein unerwünschter Viskositätsanstieg erfolgt.

**20.** System nach Anspruch 14, **dadurch gekennzeichnet, dass** die Komponente B) eine oder eine Mehrzahl von Verbindungen ist ausgewählt aus der Gruppe bestehend aus Methyl- oder Ethyltriglykolmethacrylat, Butyldiglykolmethacrylat, Tetrahydrofururylmethacrylat, Benzylmethacrylat, Isobornylmethacrylat, 1,4-Butandioldimethacrylat, Hydroxypropylmethacrylat, Trimethylolpropantrimethacrylat, Trimethacrylat eines ethoxilierten Trimethylolpropan mit 3 -10 Mol Ethylenoxid, Dimethacrylat eines ethoxilierten Bisphenol-A mit 2 -10 Mol Ethylenoxid und / oder einem Polyethylenglykoldimethacrylat mit 1 - 10 Ethylenoxid-Einheiten.

**21.** System nach Anspruch 14, **dadurch gekennzeichnet, dass** die Komponente C) Dibenzoylperoxid und/oder Dilaurylperoxid aufweist.

**22.** Verwendung eines Zwei- oder Mehrkomponentensystems nach den Ansprüchen 13 bis 21 in Klebstoffen, Gießharzen, Bodenbeschichtungen und sonstige Reaktivbeschichtungen, Abdichtmassen, Imprägniermassen, Einbettmassen, Massen für die Herstellung von künstlichem Marmor und anderen Kunststeinen, Massen für Reaktivdübel, Dentalmassen, poröse Kunststoffformen für keramische Objekte und ähnliche Anwendungen.

**23.** Verwendung eines Zwei- oder Mehrkomponentensystems nach den Ansprüchen 13 bis 21 in ungesättigten Polyesterharzen und Vinylesterharzen.

## Claims

**1.** Emulsion polymer which can be obtained by polymerization of a mixture comprising

a) from 5 to 99.9% by weight of one or more monomers having a solubility in water of < 2% by weight at 20°C and selected from the group consisting of monofunctional (meth)acrylate monomers, styrene and vinyl esters;
b) from 0 to 70% by weight of one or more monomers which can be copolymerized with the monomers a);
c) from 0 to 20% by weight of one or more doubly or multiply vinylically unsaturated compounds;
d) from 0 to 20% by weight of one or more polar monomers having a solubility in water of > 2% by weight at 20°C; and
e) 0.1 - 95% by weight of at least one activator,

with the components a) to e) adding up to 100% by weight of the polymerizable constituents of the mixture,
**characterized in that**

e1) the activator is a compound of the Formula I,

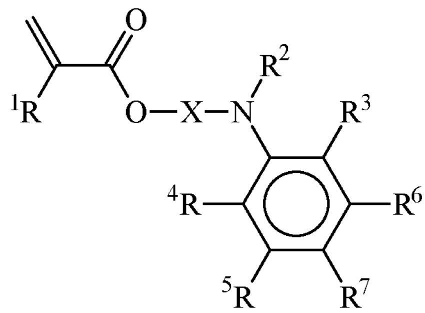

(I)

where

- $R^1$ is hydrogen or methyl;
- X is a linear or branched alkanediyl group which has from 1 to 18 carbon atoms and may be monosubstituted or polysubstituted by hydroxyl groups and/or by $C_1$ - $C_4$ alkoxy groups;
- $R^2$ is hydrogen or a linear or branched alkyl radical which has from 1 to 12 carbon atoms and may be monosubstituted or polysubstituted by hydroxyl groups or $C_1$ - $C_4$-alkoxy groups, with the hydroxyl groups being able to be partially esterified with (meth)acrylic acid;
- $R^3$, $R^4$, $R^5$, $R^6$ and $R^7$ are each, independently of one another, hydrogen or a linear or branched alkyl or alkoxy group which has from 1 to 8 carbon atoms and can be monosubstituted or polysubstituted by hydroxyl groups, where two of the radicals $R^3$ to $R^7$ may be joined to one another to form a five- to seven-membered ring and may form a fused aromatic ring system with the phenyl radical;

and **in that**
e2) the activator e) is covalently bound to the emulsion polymer.

and **in that**
the constituents a) to e) are used as core-shell polymers.

**2.** Polymer according to Claim 1, **characterized in that** $R^1$ is methyl.

**3.** Polymer according to Claim 1 or Claim 2, **characterized in that** X is an ethylene group $-CH_2-CH_2-$ a 2-hydroxypropylene group $-CH_2-CH(OH)-CH_2-$.

**4.** Polymer according to any of the preceding claims, **characterized in that** $R^2$ is selected from the group consisting of methyl, ethyl and 2-hydroxyethyl.

**5.** Polymer according to any of the preceding claims, **characterized in that** one of the radicals $R^3$ to $R^7$ is methyl while the remaining four radicals are each hydrogen or two of the radicals $R^3$ to $R^7$ are each methyl while the remaining three radicals are each hydrogen.

**6.** Polymer according to any of the preceding claims, **characterized in that** a) comprises one or more methacrylate monomers and/or acrylate monomers.

**7.** Polymer according to any of the preceding claims, **characterized in that** e) is present in an amount of 10-60% by weight, preferably 20-50% by weight.

**8.** Polymer according to Claim 6, **characterized in that** a) is methyl methacrylate.

**9.** Process for preparing an emulsion polymer, in which the constituents a) to e) according to any of Claims 1 to 8 are polymerized in aqueous emulsion.

**10.** Process according to Claim 9, in which, in the manner of a core-shell polymerization, the constituents a) to e) are polymerized as core in a first stage and a mixture of the constituents a) to d) is subsequently polymerized as shell in at least one further stage.

**11.** Process according to Claim 9, **characterized in that** the constituents a) to e) for the core and the constituents a) to d) for the shell are selected so that in the resulting polymer the glass transition temperature $T_{GS}$ of at least one shell is greater than the glass transition temperature $T_{GC}$ of the core, with the glass transition temperatures $T_G$ being determined in accordance with EN ISO 11357.

**12.** Process according to Claim 11, **characterized in that** the constituents a) to d) for the shell are selected so that in the resulting polymer the glass transition temperature $T_{GS}$ of at least one shell is greater than 100°C, with the glass transition temperature $T_{GS}$ being determined in accordance with EN ISO 11357.

**13.** Two-component or multicomponent system which comprises a polymer according to any of Claims 1 to 8 or a polymer obtained by a process according to any of Claims 9 to 12 and cures by means of a redox initiator system and has a controllable pot life.

**14.** Two-component or multicomponent system according to Claim 13 comprising

A) 0.8 - 69.94% by weight of a polymer according to any of Claims 1 to 8 or which can be obtained according to any of Claims 9 to 12;
B)30 -99.14% by weight of one or more ethylenically unsaturated monomers;
C) 0.05 - 10% by weight of peroxides; if appropriate
D) 0 - 60% by weight of unsaturated oligomers;
E) 0.01 - 2% by weight of a polymerization inhibitor; and, if appropriate,
F) 0 - 800 parts by weight of auxiliaries and additives;

with the sum of the constituents A) + B) + C) + D) + E) being 100% by weight and the amount of F) being based on 100 parts by weight of the sum of A) + B) + C) + D) + E).

**15.** System according to Claim 14 comprising
from 5 to 45% by weight of component A),
from 40 to 94.89% by weight of component B),
from 0.1 to 5% by weight of component C),
0 - 30% by weight of component D),
0.01 - 0.2% by weight of component E)
and
from 0 to 800 parts by weight of component F),
with the sum of the constituents A) + B) + C) + D) + E) being 100% by weight and the amount of F) being based on 100 parts by weight of the sum of A) + B) + C) + D) + E).

**16.** System according to Claim 14 comprising
from 5 to 45% by weight of component A),
from 50 to 94.50% by weight of component B),
from 0.5 to 5% by weight of component C),
0% by weight of component D),
and
from 0 to 800 parts by weight of component F),
with the sum of the constituents A) + B) + C) + D) + E) being 100% by weight and the amount of F) being based on 100 parts by weight of the sum of A) + B) + C) + D) + E).

**17.** System according to any of Claims 13 - 16, **characterized in that** component A) and component C) are stored together and at least one constituent of the component B) is stored separately from A) and C) until the system is used, with the swelling capability of the separately stored constituent of the component B) for the polymer A) being so high that the activator fixed to the polymer A) can react with the component C).

**18.** System according to any of Claims 13 - 16, **characterized in that** component A), part of component B) and component C) are stored together, with the proportion of the component B) being selected so that the swelling capability of this constituent of the component B) for the polymer A) is so low that the activator fixed to the polymer A) cannot react with the component C).

**19.** System according to any of Claims 13 - 16, **characterized in that** component A), and part of component B) are

stored together, with the proportion of the component B) being selected so that the swelling capability of this constituent of the component B) for the polymer A) is so low that no undesirable increase in viscosity occurs.

**20.** System according to Claim 14, **characterized in that** the component B) is one or more compounds selected from the group consisting of methyl or ethyl triglycol methacrylate, butyl diglycol methacrylate, tetrahydrofurfuryl methacrylate, benzyl methacrylate, isobornyl methacrylate, 1,4-butanediol dimethacrylate, hydroxypropyl methacrylate, trimethylolpropane trimethacrylate, the trimethacrylate of an ethoxylated trimethylolpropane containing 3 - 10 mol of ethylene oxide, the dimethacrylate of an ethoxylated bisphenol A containing 2 - 10 mol of ethylene oxide and a polyethylene glycol dimethacrylate having 1 - 10 ethylene oxide units.

**21.** System according to Claim 14, **characterized in that** the component C) comprises dibenzoyl peroxide and/or dilauryl peroxide.

**22.** Use of a two-component or multicomponent system according to any of Claims 13 to 21 in adhesives, pourable resins, floor coatings and other reactive coatings, sealing compositions, impregnation compositions, embedding compositions, compositions for producing artificial marble and other artificial stones, compositions for reactive pegs, dental compositions, porous plastic moulds for ceramic objects and similar applications.

**23.** Use of a two-component or multicomponent system according to any of Claims 13 - 21 in unsaturated polyester resins and vinyl ester resins.

## Revendications

**1.** Polymère en émulsion pouvant être obtenu par polymérisation d'un mélange, présentant

a) 5% en poids à 99,9% en poids d'un ou de plusieurs monomères présentant une solubilité dans l'eau < 2% en poids à 20°C, choisis dans le groupe constitué par les monomères de (méth)acrylate monofonctionnels, le styrène et les esters de vinyle ;
b) 0% en poids à 70% en poids d'un ou de plusieurs monomères copolymérisables avec les monomères a) ;
c) 0% en poids à 20% en poids d'un ou de plusieurs composés vinyliquement diinsaturés ou polyinsaturés ;
d) 0% en poids à 20% en poids d'un ou de plusieurs monomères polaires présentant une solubilité dans l'eau > 2% en poids à 20°C ; et
e) 0,1-95% en poids d'au moins un activateur,

les composants a) à e) représentant ensemble 100% en poids des constituants polymérisables du mélange, **caractérisé en ce que**

e1) l'activateur est un composé de formule I,

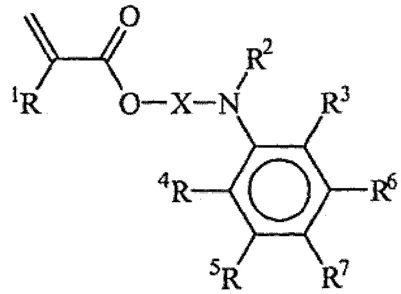

**(I)**

où

$R^1$ représente hydrogène ou méthyle ;
X représente un groupe alcanediyle linéaire ou ramifié, comprenant 1 à 18 atomes de carbone, qui peut être monosubstitué ou polysubstitué par des groupes hydroxyle et/ou des groupes $C_1$-$C_4$-alcoxy ;

$R^2$ signifie hydrogène ou un radical alkyle linéaire ou ramifié comprenant 1 à 12 atomes de carbone, qui est le cas échéant monosubstitué ou polysubstitué par des groupes hydroxyle ou des groupes $C_1$-$C_4$-alcoxy, les groupes hydroxyle pouvant être estérifiés partiellement par de l'acide (méth)acrylique ;
$R^3$, $R^4$, $R^5$, $R^6$ et $R^7$ signifient, indépendamment l'un de l'autre, hydrogène ou un groupe alkyle ou alcoxy linéaire ou ramifié, comprenant 1 à 8 atomes de carbone, qui peuvent être monosubstitués ou polysubstitués par des groupes hydroxyle ; et le cas échéant deux des radicaux $R^3$ à $R^7$ étant liés l'un à l'autre en un cycle de cinq à sept chaînons et formant le cas échéant un système cyclique aromatique condensé avec le radical phényle ; et **en ce que**

e2) l'activateur e) est incorporé dans le polymère en émulsion via des liaisons covalentes et **en ce que** les constituants a) à e) sont utilisés sous forme de polymères à noyau-coquille.

**2.** Polymère selon la revendication 1, **caractérisé en ce que** $R^1$ représente méthyle.

**3.** Polymère selon la revendication 1 ou la revendication 2, **caractérisé en ce que** X représente un groupe éthylène $-CH_2-CH_2-$ ou un groupe 2-hydroxypropylène $-CH_2-CH(OH)-CH_2-$.

**4.** Polymère selon l'une quelconque des revendications précédentes, caractérisé en ce $R^2$ est choisi dans le groupe constitué par méthyle, éthyle et 2-hydroxyéthyle.

**5.** Polymère selon l'une quelconque des revendications précédentes, **caractérisé en ce qu'**un des radicaux $R^3$ à $R^7$ représente méthyle alors que les quatre autres radicaux représentent hydrogène ou deux des radicaux $R^3$ à $R^7$ représentent méthyle alors que les trois autres radicaux représentent hydrogène.

**6.** Polymère selon l'une quelconque des revendications précédentes, **caractérisé en ce que** a) est constitué par un ou plusieurs monomères de méthacrylate et/ou d'acrylate.

**7.** Polymère selon l'une quelconque des revendications précédentes, **caractérisé en ce que** e) se trouve en une quantité de 10-60% en poids, de préférence de 20-50% en poids.

**8.** Polymère selon la revendication 6, **caractérisé en ce que** a) représente du méthacrylate de méthyle.

**9.** Procédé pour la préparation d'un polymère en émulsion, dans lequel on polymérise les constituants a) à e) selon les revendications 1 à 8 dans une émulsion aqueuse.

**10.** Procédé selon la revendication 9, dans lequel on polymérise, selon un type de polymérisation à noyau-coquille, les constituants a) à e), dans une première étape, sous forme de noyau, puis dans au moins une autre étape, comme coquille, un mélange des constituants a) à d).

**11.** Procédé selon la revendication 9, **caractérisé en ce qu'**on choisit les constituants a) à e) pour le noyau et les constituants a) à d) pour la coquille de manière telle que dans le polymère résultant, la température de transition vitreuse $T_{GC}$ d'au moins une coquille est supérieure à la température de transition vitreuse $T_{GN}$ du noyau, les températures de transition vitreuse $T_G$ étant déterminées selon la norme EN ISO 11357.

**12.** Procédé selon la revendication 11, **caractérisé en ce qu'**on choisit les constituants a) à d) pour la coquille de manière telle que dans le polymère résultant, la température de transition vitreuse $T_{GC}$ d'au moins une coquille est supérieure à 100°C, la température de transition vitreuse $T_{GC}$ étant déterminée selon la norme EN ISO 11357.

**13.** Système à deux ou à plus de deux composants, durcissant sous l'effet d'un système initiateur redox, présentant une durée de vie en pot contrôlable, présentant un polymère selon les revendications 1 à 8 ou un polymère obtenu selon un procédé selon les revendications 9 à 12.

**14.** Système à deux ou à plus de deux composants selon la revendication 13, comprenant

A) 0,8-69,94% en poids d'un polymère selon les revendications 1 à 8 ou pouvant être obtenu selon le procédé selon les revendications 9 à 12 ;
B) 30 à 99,14% en poids d'un ou de plusieurs monomères éthyléniquement insaturés ;
C) 0,05-10% en poids de peroxydes ; le cas échéant

D) 0-60% en poids d'oligomères insaturés ;
E) 0,01-2% en poids d'un inhibiteur de polymérisation ; et le cas échéant
F) 0-800 parties en poids d'adjuvants et d'additifs ;

la somme des constituants A) + B) + C) + D) + E) valant 100% en poids et la quantité de F) se rapportant à 100 parties en poids de la somme de A) + B) + C) + D) + E).

**15.** Système selon la revendication 14, contenant 5 à 45% en poids de composant A),

40 à 94,89% en poids de composant B),
0,1 à 5% en poids de composant C),
0 à 30% en poids de composant D) ;
0,01 à 0,2% en poids de composant E) et
0 à 800 parties en poids de composant F),

la somme des constituants A) + B) + C) + D) + E) valant 100% en poids et la quantité de F) se rapportant à 100 parties en poids de la somme de A) + B) + C) + D) + E).

**16.** Système selon la revendication 14, contenant

5 à 45% en poids de composant A),
50 à 94,50% en poids de composant B),
0,5 à 5% en poids de composant C),
0% en poids de composant D) ; et
0 à 800 parties en poids de composant F),

la somme des constituants A) + B) + C) + D) + E) valant 100% en poids et la quantité de F) se rapportant à 100 parties en poids de la somme de A) + B) + C) + D) + E).

**17.** Système selon l'une quelconque des revendications 13 à 16, **caractérisé en ce que** le composant A) et le composant C) sont entreposés ensemble et, jusqu'à l'utilisation du système, au moins un constituant du composant B) est entreposé séparément de A) et C), le pouvoir gonflant du constituant entreposé séparément du composant B) pour le polymère A) étant tellement élevé que l'activateur fixé sur le polymère du polymère A) peut se transformer avec le composant C).

**18.** Système selon l'une quelconque des revendications 13 à 16, **caractérisé en ce que** le composant A), une partie du composant B) et le composant C) sont entreposés ensemble, la partie du composant B) étant choisie de manière telle que le pouvoir gonflant de ce constituant du composant B) pour le polymère A) est tellement bas que l'activateur fixé sur le polymère du polymère A) ne peut pas se transformer avec le composant C).

**19.** Système selon l'une quelconque des revendications 13 à 16, **caractérisé en ce que** le composant A) et une partie du composant B) sont entreposés ensemble, la partie du composant B) étant choisie de manière telle que le pouvoir gonflant de ce constituant du composant B) pour le polymère A) est tellement bas qu'une augmentation non souhaitée de la viscosité ne se produit pas.

**20.** Système selon la revendication 14, **caractérisé en ce que** le composant B) est formé par un ou plusieurs composés, choisis dans le groupe constitué par le méthacrylate de méthyltriglycol ou d'éthyltriglycol, le méthacrylate de butyldiglycol, le méthacrylate de tétrahydrofurfuryle, le méthacrylate de benzyle, le méthacrylate d'isobornyle, le diméthacrylate de 1,4-butanediol, le méthacrylate d'hydroxypropyle, le triméthacrylate de triméthylolpropane, le triméthacrylate d'un triméthylolpropane éthoxylé par 3-10 moles d'oxyde d'éthylène, le diméthacrylate d'un bisphénol-A éthoxylé par 2-10 moles d'oxyde d'éthylène et/ou un diméthacrylate de polyéthylène présentant 1-10 unités d'oxyde d'éthylène.

**21.** Système selon la revendication 14, **caractérisé en ce que** le composant C) présente du peroxyde de dibenzoyle et/ou du peroxyde de dilauryle.

**22.** Utilisation d'un système à deux ou plus de deux composants selon les revendications 13 à 21 dans des adhésifs, des résines coulées, des revêtements de sol et d'autres revêtements réactifs, des masses de bouchage, des masses

d'imprégnation, des masses d'enrobage, des masses pour la préparation de marbre synthétique et d'autres pierres synthétiques, des masses pour des chevilles réactives, des masses dentaires, des moules synthétiques poreux pour objets céramiques et des utilisations analogues.

**23.** Utilisation d'un système à deux ou plus de deux composants selon les revendications 13 à 21 dans des résines de polyester insaturées et des résines d'ester de vinyle insaturées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4315788
- DE 1544924
- DE 2710548
- DE 10051762
- WO 9915592 A
- DE 10339329 A1
- EP 0376096 B1